# EUROPEAN PATENT APPLICATION

(11) **EP 4 382 135 A1**
(43) Date of publication of application: **12.06.2024**
(21) Application number: 22853581.1
(22) Date of filing: 08.08.2022
(51) Int. Cl.: A61K 48/00, A61K 31/7105, A61P 35/00, C12N 15/113

(54) **CANCER-SPECIFIC TRANS-SPLICING RIBOZYME AS GENE THERAPY PRODUCT COMBINED WITH RADIOTHERAPY**

(30) Priority: 06.08.2021 KR 20210103814; 05.08.2022 KR 20220098089
(71) Applicant: RZNOMICS INC., Yongin-si, Gyeonggi-do 16890 (KR)
(72) Inventor: LEE, Seong-Wook, Seoul 02780 (KR); HAN, Seung Ryul, Yongin-si, Gyeonggi-do 16919 (KR); PARK, Hee Chul, Seoul 06355 (KR); CHOI, Changhoon, Seongnam-si, Gyeonggi-do 13600 (KR); SHIN, Sungwon, Seoul 05841 (KR)
(74) Representative: De Clercq & Partners
(86) International application number: PCT/KR2022/011774
(87) International publication number: WO 2023/014206

(57) **Abstract**

The present invention relates to a cancer-specific trans-splicing ribozyme as a gene therapy product combined with radiotherapy. The ribozyme of the present disclosure is safe because of the cancer tissue-specific expression thereof and exhibits high expression efficiency at a post-transcriptional level and thus can be provided as a safe and effective gene therapy product. When administered after radiotherapy, the ribozyme exhibits high anticancer effects even at a low dose of radiation and a low dose of gene therapy product and thus is expected to be used in a cancer therapy method and a radiation-resistant cancer therapy method with no or little adverse effects.

## Description

### Technical Field

The present disclosure relates to a cancer-specific trans-splicing ribozyme as a gene therapy product combined with radiotherapy, and the like.

### Background Art

Telomerase is a ribonucleoprotein that restores the telomere region that is shortened during DNA replication by repeatedly adding a TTAGGG sequence to the end of the telomere present at the 3' end of the chromosome to enable the immortal proliferation of cells. The telomerase is one of the most important enzymes that regulate the immortality and proliferation ability of cancer cells. While hematopoietic cells and 80 to 90% of cancer cells have telomerase activity, normal cells near cancer cells have no telomerase activity, and furthermore, telomerase reactivation has a major impact on the immortal growth of advanced metastatic cancer.

Human telomerase consists of two components of human telomerase RNA (hTR) serving as a substrate RNA, and human telomerase reverse transcriptase (hTERT) serving as a catalyst. A human hTERT gene is expressed in proportion to telomerase activity, and there is a strong correlation between intracellular hTERT levels and cellular telomerase activity. In particular, TERT activity is observed in 90% or more of cancer patients.

Recently, as a trans-splicing ribozyme targeting this hTERT has become known, attempts to develop a cancer therapy product using the trans-splicing ribozyme have been actively conducted. However, while a combination of the trans-splicing ribozyme and a tissue-specific promoter shows high tissue specificity, the expression efficiency is very low, which is a disadvantage problem in terms of therapeutic efficiency. In addition, since the telomerase activity is also present even in normal cells such as stem cells, hematopoietic stem cells, germ cells, and regenerating normal liver cells, treatment targeting hTERT may cause toxicity to these cells. Although liver cells are weak, 5% of normal liver cells have telomerase activity, and a regenerating liver is known to have increased telomerase activity. In particular, hepatocellular carcinoma (HCC) is mostly accompanied by cirrhosis, and TERT is expressed, albeit at a low level, in non-tumorous hepatocytes that constitute regenerative nodules in these cirrhotic areas.

Korean Patent Publication No. 10-2016-0038674 discloses a recombinant vector in which a nucleic acid sequence recognizing microRNA-122 (miR-122) is additionally linked to a ribozyme-target gene expression cassette including a tissue-specific promoter, a trans-splicing ribozyme targeting a cancer-specific gene, and a target gene linked to a 3' exon of the ribozyme, and a use for preventing or treating liver cancer of the ribozyme expressed from the recombinant vector. The miR-122 is a microRNA known to be highly overexpressed in normal liver, and its expression decreases when advanced to liver cancer. In the prior arts, based on this phenomenon, a miR-122 target site is introduced into a 3' UTR site of a ribozyme expression vector, so that the ribozyme delivered to the liver is not expressed by overexpressed miR-122 in the normal liver, but may be expressed and act in the liver cancer with the decreased miR-122 level.

Meanwhile, radiotherapy is an effective method for cancer therapy and has been widely used for various tumors, but has problems in that the upper limit of a dose of radiation is limited due to a problem of causing damage to surrounding normal tissues, and a phenomenon occurs in which cancer cells acquire radiation resistance and immunosuppressive effects due to repeated radiotherapy at a low dose of radiation. In particular, there is a problem in radiotherapy for liver cancer that there is a limitation in radiotherapy and a dose due to the proximity to major organs of the human body.

As a recent gene therapy method, research on the combination of various therapy methods is active to resolve a problem of high cost and a problem of requiring treatment of vector-introduced virus at a high concentration of the related art in order to show a therapeutic effect, reduce the adverse effects of radiotherapy, and overcome the limitations.

### Disclosure of the Invention

### Technical Goals

The present disclosure is intended to solve the above problems, and an aspect of the present disclosure is to provide a cancer-specific trans-splicing ribozyme with excellent safety and expression efficiency as a gene therapy product combined with radiotherapy in order to overcome adverse effects and limitations of radiotherapy.

However, technical goals to be achieved are not limited to those described above, and other goals not mentioned above are clearly understood by one of ordinary skill in the art from the following description.

### Technical Solutions

The present inventors made intensive research on a gene therapy method to resolve adverse effects and limitations of cancer immunotherapy, targeted therapy, and radiotherapy, and as a result, confirmed that a trans-splicing ribozyme targeting a cancer-specific gene sequence in cancer cells was expressed or administered in combination with the cancer immunotherapy, targeted therapy, and radiotherapy to reduce adverse effects of each therapy method and improve a cancer therapy effect, and then completed the present disclosure.

The present disclosure provides a pharmaceutical composition for preventing or treating cancer, including a trans-splicing ribozyme expression vector targeting a cancer-specific gene sequence, a gene delivery system including the vector, and/or a ribozyme expressed from the vector, and combined with radiotherapy.

Further, the present disclosure provides a pharmaceutical composition for enhancing a radiotherapy effect of cancer, including the trans-splicing ribozyme expression vector targeting the cancer-specific gene sequence, the gene delivery system including the vector, and/or the ribozyme expressed from the vector.

Further, the present disclosure provides a pharmaceutical composition for treating radiation-resistant cancer, including a trans-splicing ribozyme expression vector targeting a cancer-specific gene sequence, a gene delivery system including the vector, or a ribozyme expressed from the vector as an active ingredient.

As an embodiment of the present disclosure, the expression vector may include a cytomegalovirus (CMV) promoter operably linked to the ribozyme gene, a splicing donor/splicing acceptor sequence (SD/SA sequence) at the 5' end site of the ribozyme gene, and a Woodchuck hepatitis virus posttranscriptional regulatory element (WPRE) at the 3' end site.

As another embodiment of the present disclosure, the expression vector may include a target gene linked to the 3' exon of the ribozyme gene.

As yet another embodiment of the present disclosure, the expression vector may include a gene encoding a sequence complementary to part or all of microRNA-122 (miR-122) at the 3'-UTR end site of the ribozyme gene.

As yet another embodiment of the present disclosure, the cancer-specific gene sequence is essential for the growth, proliferation, and/or metastasis of cancer cells, and is not limited as long as the gene sequence is any gene overexpressed in cancer cells compared to normal cells. Desirably, the cancer-specific gene sequence may be at least one gene selected from the group consisting of telomerase reverse transcriptase (TERT) mRNA, alpha fetoprotein (AFP) mRNA, carcinoembryonic antigen (CEA) mRNA, prostate-specific antigen (PSA) mRNA, cytoskeleton-associated protein 2 (CKAP2) mRNA, and mutant rat sarcoma (RAS) mRNA.

As yet another embodiment of the present disclosure, the TERT mRNA sequence may consist of or include a base sequence represented by SEQ ID NO: 2.

As yet another embodiment of the present disclosure, the trans-splicing ribozyme may consist of or include a base sequence represented by SEQ ID NO: 3.

As yet another embodiment of the present disclosure, the target gene may be an anti-cancer therapeutic gene or reporter gene, and the anti-cancer therapeutic gene may be at least one gene selected from the group consisting of a drug-sensitizing gene, a proapoptotic gene, a cytostatic gene, a cytotoxic gene, a tumor suppressor gene, an antigenic gene, a cytokine gene, and an anti-angiogenic gene, and the drug-sensitizing gene may be a Herpes Simplex Virus thymidine kinase (HSVtk) gene.

As yet another embodiment of the present disclosure, the HSVtk gene may consist of or include a base sequence represented by SEQ ID NO: 4.

As yet another embodiment of the present disclosure, the reporter gene may be luciferase, green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescence protein (EYFP), cyan fluorescent protein (CFP) or enhanced cyan fluorescent protein (ECFP).

As yet another embodiment of the present disclosure, the gene encoding a sequence complementary to miR-122 may express a sequence complementary to part or all of miR-122, desirably express a sequence complementary to all of miR-122, but is not limited to substitution, deletion or insertion of some bases as long as the sequence binds to intracellular miR-122 to induce the ribozyme degradation of the present disclosure. In the present disclosure, the gene encoding the sequence complementary to miR-122 may consist of or include a base sequence represented by SEQ ID NO: 5, and may repeatedly include a base sequence represented by SEQ ID NO: 5.

As yet another embodiment of the present disclosure, the gene delivery system may be a viral vector including the expression vector, and the viral vector may be an adenovirus vector.

As yet another embodiment of the present disclosure, the cancer to be prevented or treated of the present disclosure is not limited as long as the cancer is any disease in a modified state caused by abnormal proliferation of cells. Non-limiting examples thereof include liver cancer, thyroid cancer, testicular cancer, bone cancer, glioblastoma, ovarian cancer, brain cancer, gallbladder cancer, biliary tract cancer, colon cancer, head and neck cancer, lymphoma, bladder cancer, leukemia, peritoneal cancer, small intestine cancer, esophageal cancer, renal pelvis cancer, kidney cancer, heart cancer, duodenal cancer, eye cancer, urethral cancer, breast cancer, stomach cancer, prostate cancer, uterine cancer, lung cancer, spinal cord tumor, pancreatic cancer, melanoma, and the like. Meanwhile, the cancer may be desirably cancer in which miR-122 is not substantially expressed in the cancer tissue, and specifically, cancer in which the number of copies of miR-122 in the cancer tissue is 100 times less than the number of copies of the ribozyme expressed in the cancer tissue by the pharmaceutical composition.

As yet another embodiment of the present disclosure, the liver cancer is not limited to the etiology, and non-limiting examples of the etiology include hepatitis B virus, hepatitis C virus, in which the expression of miR-122 is reduced in liver cancer tissue, alcohol, chronic hepatitis, cirrhosis, non-alcoholic fatty liver, aflatoxin, family history, and the like.

As yet another embodiment of the present disclosure, the pharmaceutical composition may be administered by a route selected from the group consisting of intravenous, intraarterial, intracancer tissue, and subcutaneous routes, and may be administered in the form of injections.

As yet another embodiment of the present disclosure, the pharmaceutical composition may be administered simultaneously or sequentially with radiotherapy. Desirably, the pharmaceutical composition may be administered after radiotherapy. More desirably, the pharmaceutical composition may be administered several times a day simultaneously, sequentially, or separately with radiotherapy after radiotherapy 1 to 3 times.

In addition, the present disclosure provides a cancer therapy method, including administering to a subject a trans-splicing ribozyme expression vector targeting a cancer-specific gene sequence, a gene delivery system including the vector, or a ribozyme expressed from the vector in combination with radiotherapy.

As an embodiment of the present disclosure, the administering step combined with radiotherapy may be performed simultaneously with or sequentially with radiotherapy, but desirably performed after radiotherapy.

More specifically, the cancer therapy method of the present disclosure may include the following steps:
(1) performing radiotherapy on a subject in need of cancer therapy; and
(2) administering to the subject a trans-splicing ribozyme expression vector targeting a cancer-specific gene sequence, a gene delivery system including the vector, or a ribozyme expressed from the vector simultaneously with radiotherapy.

As an embodiment of the present disclosure, in the cancer therapy method, step 2 may be performed after step 1, and step 1 may be performed after step 2. In other words, the order of steps 1 and 2 above does not matter.

As another embodiment of the present disclosure, steps 1 and 2 may be independently repeated several times.

As another embodiment of the present disclosure, step 2 may be performed simultaneously when step 1 is performed once, and first simultaneously with the same day as the last step 1 when step 2 is performed two or more times.

As another embodiment of the present disclosure, when steps 1 and 2 are each performed twice or more, step 1 may be performed every day, and step 2 may be performed every other day.

As another embodiment of the present disclosure, when steps 1 and 2 are each performed twice or more, step 2 may be performed first on the day when the last step 1 is performed.

As another embodiment of the present disclosure, step 1 may be irradiating the subject with radiation of 1 to 4 Gy, desirably 1.5 to 2.5 Gy, and more desirably 2 Gy.

As another embodiment of the present disclosure, the subject is not limited to any mammal in need of cancer therapy, but may be desirably humans.

In addition, the present disclosure provides a use of the trans-splicing ribozyme expression vector targeting a cancer-specific gene sequence, the gene delivery system including the vector, or the ribozyme expressed from the vector for preparing a cancer therapy drug combined with radiotherapy.

### Effects

According to the present disclosure, the trans-splicing ribozyme does not act on normal tissues, but is specifically expressed in cancer tissues to have high safety and excellent expression efficiency at a post-transcriptional level, and thus can be effectively used to treat cancer. In addition, according to the present disclosure, the ribozyme exhibits an increased cancer therapy effect in combination with other cancer therapy methods and can be used as a drug for the treatment of carcinoma resistant to radiotherapy. Particularly, when combined with radiotherapy, the ribozyme exhibits high anticancer effects even at a low dose of radiation and a low dose of gene therapy product and thus can reduce adverse effects and increase accessibility to treatment.

### Brief Description of Drawings

FIG. 1 is a structure of a CMV promoter-based hTERT targeting trans-splicing ribozyme and a target gene expression cassette of the present disclosure and a schematic view of an experiment of confirming an anticancer effect of adenovirus according to radiation-combined treatment.
FIG. 2 is a graph showing (a) tumor size and (b) tumor weight confirmed by injecting a glioblastoma cell line LN229 into nude mice to induce tumor formation and then administering ECRT-122T adenovirus.
FIG. 3 is a graph showing tumor size confirmed by injecting a glioblastoma cell line U87MG into nude mice to induce tumor formation and then administering ECRT-122T adenovirus.
FIG. 4 is a graph showing a result of measuring AST and ALT levels, after injecting ECRT-122T adenovirus into normal ICR mice.
FIG. 5 is a graph showing a result of measuring body weight (a), feed consumption (b), and liver weight (c) of mice, after injecting ECRT-122T adenovirus into normal ICR mice.
FIG. 6 illustrates a result of histopathological test of the liver performed after injecting different doses of ECRT-122T adenovirus into normal ICR mice.
FIG. 7 is a graph showing a result of measuring AST and ALT levels, after injecting ECRT-122T adenovirus into normal ICR mice and then treating GCV.
FIG. 8 is a graph showing a result of measuring body weight (a), feed consumption (b), and liver weight (c) of mice, after injecting ECRT-122T adenovirus into normal ICR mice and then treating GCV.
FIG. 9 illustrates results of histopathological test of the liver performed after injecting different doses of ECRT-122T adenovirus into normal ICR mice and then treating GCV.
FIG. 10 illustrates a result of comparing anticancer effects by injecting SNU398 cells into a mouse xenograft subcutaneous model to induce tumor formation and then administering CRT-122T or ECRT-122T adenovirus.
FIG. 11 is a schematic view of an experiment of confirming the anticancer effect of adenovirus according to radiation-combined treatment.
FIG. 12 illustrates a result of comparing changes in body weight of mice according to administration of adenovirus and combined radiation treatment, after injecting SNU398 cells into a mouse xenograft subcutaneous model to induce tumor formation.
FIG. 13 illustrates a result of comparing changes in size of tumor tissue according to administration of adenovirus and combined radiation treatment, after injecting SNU398 cells into a mouse xenograft subcutaneous model to induce tumor formation.
FIG. 14 illustrates a result of comparing changes in size of tumor tissue according to administration of adenovirus and combined radiation treatment, after injecting Hep3B cells into a mouse xenograft subcutaneous model to induce tumor formation.

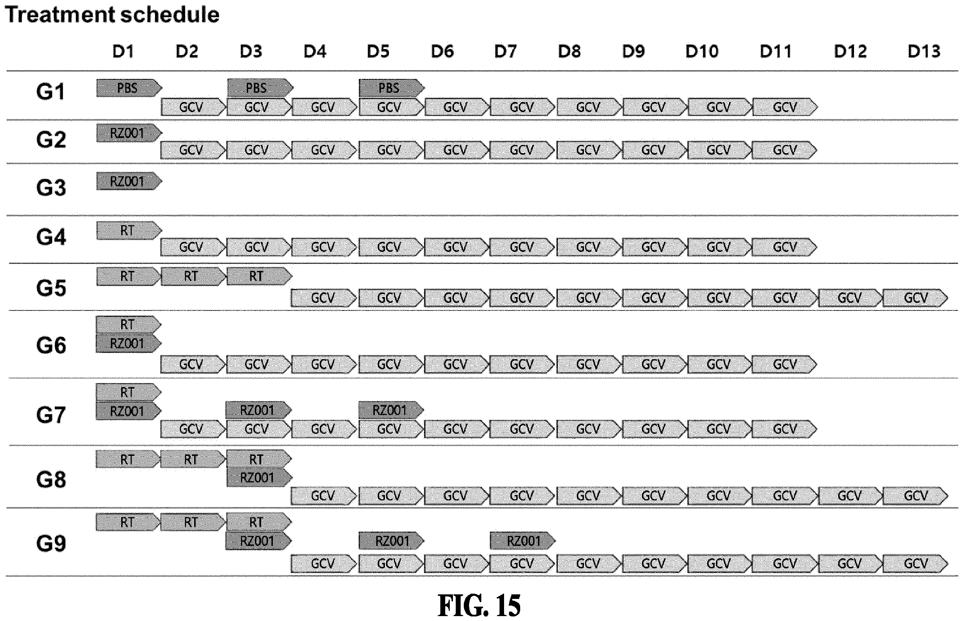

FIG. 15 is a schematic view of an experiment for establishing an optimal adenovirus and radiation-combined treatment protocol.
FIG. 16A illustrates a result of comparing changes in size of tumor tissue, after injecting SNU398 cells into a mouse xenograft subcutaneous model to induce tumor formation and then treating different methods and doses of adenovirus administration and/or irradiation.
FIG. 16B illustrates a result of comparing changes in body weight of mice, after injecting SNU398 cells into a mouse xenograft subcutaneous model to induce tumor formation and then treating different methods and doses of adenovirus administration and/or irradiation.
FIG. 17 illustrates a result of confirming the expression levels of rH2AX and p-ATM through immunofluorescence staining at 4 hours and 48 hours after irradiation and/or RZ-001 infection in a Hep3B human liver cancer cell line.

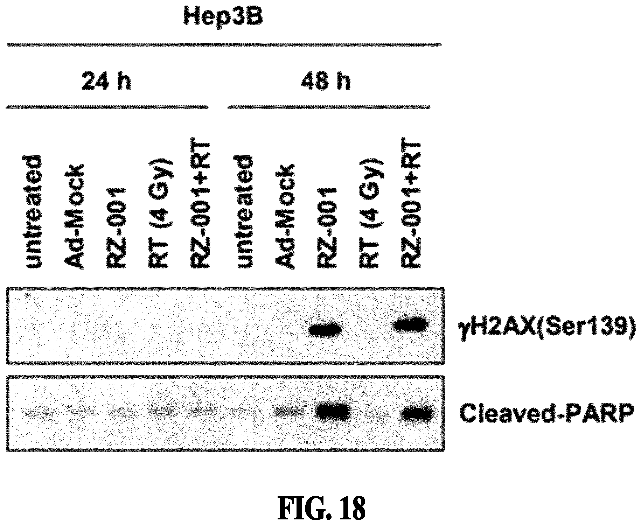

FIG. 18 illustrates a result of confirming the expression levels of rH2AX and cleaved-PARP through western blotting at 24 hours and 48 hours after irradiation and/or RZ-001 infection in a Hep3B human liver cancer cell line.

### Best Mode for Carrying Out the Invention

The present disclosure may have various modifications and various Examples, and specific Examples will be hereinafter illustrated in the drawings and described in detail in the detailed description. However, the present disclosure is not intended to be limited to specific Examples, and it should be understood that the present disclosure covers all the modifications, equivalents and replacements within the idea and technical scope of the present disclosure. In the interest of clarity, not all details of the relevant art are described in detail in the present specification in so much as such details are not necessary to obtain a complete understanding of the present disclosure.

### [Examples]

Hereinafter, the present disclosure will be described in more detail.

In the present disclosure, when a certain part "comprises" a certain component, this means that the part may further include another component without excluding another component unless otherwise stated.

### [Recombinant Vector]

An aspect of the present disclosure provides a recombinant vector including:
(i) a cytomegalovirus promoter; and
(ii) a ribozyme-target gene expression cassette including a trans-splicing ribozyme targeting a cancer-specific gene sequence and a target gene linked to the 3' exon of the ribozyme,
   in which in the expression cassette, a splicing donor/splicing acceptor sequence (SD/SA sequence) is linked to the 5' end of the ribozyme-target gene expression cassette, and a WPRE is linked to the 3' end, and
(iii) a nucleic acid sequence recognizing microRNA-122 is additionally linked to the 3' end of the WPRE.

In the present disclosure, the recombinant vector is named ECRT-122T.

The cytomegalovirus (CMV) promoter included in the recombinant vector according to the present disclosure may increase the ribozyme expression in Hep3B, SNU398, and SNU449 cell lines compared to a PEPCK promoter. In addition, the *in-vivo* expression efficiency of the ribozyme of the recombinant vector is higher when the SD/SA sequence and the WPRE are included as components at both ends of the ribozyme and the target gene, together with the CMV promoter. Based on this, it is possible to treat various cancer cells as well as liver cancer cells by simultaneously using the CMV promoter, the SD/SA sequence, and the WPRE, and additionally including a nucleic acid sequence miR-122T that recognizes miR-122.

As used herein, the term "vector" is an expression vector capable of expressing a target gene in a suitable host cell, and refers to a gene construct containing an essential regulatory element operably linked to express a gene insert contained in the vector.

As used herein, the term "operably linked" means that a nucleic acid sequence encoding a target gene is functionally linked to a nucleic acid expression regulatory sequence that performs a general function.

For example, when a ribozyme coding sequence is operably linked to a promoter, the expression of the ribozyme coding sequence is under the influence or control of the promoter. Two nucleic acid sequences (a ribozyme coding sequence and a promoter site sequence at the 5' end of the ribozyme coding sequence) are operably linked when the ribozyme coding sequence is transcribed by inducing the action of the promoter, and the linkage characteristic between the two sequences does not induce a frameshift mutation, and the expression regulatory sequence may be considered to be operably linked if the expression of the ribozyme is not inhibited. The operable linkage with the recombinant vector may be prepared using a gene recombination technique well-known in the art, and site-specific DNA cleavage and linkage may be performed by using enzymes and the like which are generally known in the art.

The vector according to the present disclosure includes a signal sequence or a leader sequence for membrane targeting or release in addition to expression regulatory elements such as a promoter, an operator, an initiation codon, a termination codon, a polyadenylation signal, and an enhancer, and may be variously prepared according to a purpose. The promoter of the vector may be constitutive or inductive. Further, the expression vector includes a selective marker for selecting a host cell containing a vector and a replicable expression vector may include a replication origin. The vector may be self-replicated or integrated with host DNA.

The vector according to the present disclosure may be desirably a plasmid vector, a cosmid vector, a viral vector or the like, and most desirably a viral vector. The viral vector may be desirably vectors derived from retrovirus, such as human immunodeficiency virus (HIV), murine leukemia virus (MLV), avian sarcoma/leucosis virus (ASLV), spleen necrosis virus (SNV), Rous sarcoma virus (RSV), and mouse mammary tumor virus (MMTV), adenovirus, adeno-associated virus (AAV), herpes simplex virus (HSV), or the like, but is not limited thereto. The recombinant vector according to the present disclosure may be most desirably a recombinant adenoviral vector.

As used herein, the term "expression cassette" means a unit cassette capable of expressing a trans-splicing ribozyme-target gene that includes a CMV promoter and a trans-splicing ribozyme-target gene, in which an SD/SA sequence and a WPRE sequence are present at each end of the trans-splicing ribozyme-target gene, and a nucleic acid sequence recognizing miR-122 is additionally linked to the 3' end of the WPRE.

The trans-splicing ribozyme-target gene expression cassette according to the present disclosure may further include a sequence that regulates the transcription level, that is, a regulatory derivative, in a sequence in which the trans-splicing ribozyme and the target gene are linked. It is not limited thereto, but in the present disclosure, particularly, a splicing donor/splicing acceptor sequence (SD/SA sequence) and/or a Woodchuck hepatitis virus Posttranscriptional Regulatory Element (WPRE) are linked, and a sequence recognizing miR-122 may be additionally linked to the 3' end of the WPRE. As a result, the expression level of the ribozyme-target gene may be regulated, and the ribozyme is expressed only when miR-122 is below a certain level, thereby minimizing the impact on normal liver cells.

In the ribozyme-target gene expression cassette according to the present disclosure, desirably, the SD/SA sequence may be linked to the 5' end of the ribozyme, the WPRE may be linked to the 3' end of the target gene, and a sequence recognizing miR-122 may be linked to the 3' end of the WPRE.

The SD/SA according to the present disclosure increases transcription initiation, processing of RNA polymerase II, and a nucleocytoplasmic export of mRNA, and the WPRE according to the present disclosure may increase a level of pre-mRNA by increasing the processing and the nucleocytoplasmic export of mRNA, respectively. Through the configuration, an RNA level of the ribozyme is significantly increased in the cell to allow cancer cell-specific expression while increasing the death of cancer cells *in vivo,* so that toxicity to normal cells may be rather reduced.

The SD/SA sequence according to the present disclosure is a sequence corresponding to a starting part and an end part of an intron that is cleaved in a splicing reaction of removing the intron of an RNA transcript, and generally, the SD sequence may be a GU sequence at the 5' end of the intron, and the SA sequence may be an AG sequence at the 3' end of the intron.

The Woodchuck Hepatitis Virus (WHP) Posttranscriptional Regulatory Element (WPRE) according to the present disclosure refers to a sequence that increases gene expression by inducing a tertiary structure in DNA that promotes transcription.

In the present disclosure, the SD/SA sequence and the WPRE sequence may include sequences represented by SEQ ID NO: 6 and SEQ ID NO: 7, respectively, but are not limited thereto as long as the sequences are present in the target gene expression cassette to promote the expression of the target gene.

The nucleic acid sequence recognizing miR-122 according to the present disclosure is referred to herein as a microRNA-122 target site (miR-122T). The miR-122T may include a sequence represented by SEQ ID NO: 5 repeated one or more times, for example, 1 to 10 times, desirably 1 to 5 times, and more desirably 1 to 3 times. The miR-122 is normally expressed in normal liver cells, but the expression level thereof is reduced in liver cancer cells. Using this, it is possible to develop a therapy product with increased sensitivity and specificity to liver cancer cells, and in the present disclosure, a nucleic acid sequence recognizing miR-122 is linked to a ribozyme to which a target gene is linked to perform the expression of a liver cancer cell-specific ribozyme.

As used herein, the term "cancer-specific gene" refers to a gene that is specifically expressed or remarkably overexpressed only in cancer cells. The cancer-specific gene may add a feature that the ribozyme according to the present disclosure acts in a cancer-specific manner. Such a cancer-specific gene may be desirably telomerase reverse transcriptase (TERT) mRNA, alpha fetoprotein (AFP) mRNA, carcinoembryonic antigen (CEA) mRNA, prostate-specific antigen (PSA) mRNA, cytoskeleton-associated protein 2 (CKAP2) mRNA, or mutant Rat sarcoma (RAS) mRNA, more desirably telomerase reverse transcriptase (TERT) mRNA, and most desirably a human telomerase reverse transcriptase (hTERT) mRNA sequence. The ribozyme of the present disclosure includes the cancer-specific gene to exhibit anticancer effects even in cancer cell lines of a tissue that does not substantially express miR-122. The present inventors have found that apoptosis increases when treating adenovirus expressing ECRT-122T, particularly to a glioblastoma cell line, a colon cancer cell line, a melanoma cell line, a cervical cancer cell line, a lung cancer cell line, an osteosarcoma cell line, a breast cancer cell line, and a biliary tract cancer cell line.

As used herein, the term "telomerase reverse transcriptase (TERT)" is one of the most important enzymes that regulate the immortality and proliferation ability of cancer cells, and refers to an enzyme that inhibits the aging of cells by forming a telomere structure on the chromosome to protect the chromosome end. In normal cells, whenever the cells divide, the length of the telomere decreases little by little, and eventually, a genetic substance is lost and the cells die. However, in cancer cells, this enzyme continuously extends the telomere, so that the cells do not die, and the enzyme directly contributes to the immortality of cancer cells, which is known as a major obstacle to treat cancer. In the present disclosure, hTERT mRNA including a sequence represented by SEQ ID NO: 2 may be used as a cancer-specific gene, but is not limited thereto.

As used herein, the term "promoter" is involved in the binding of RNA polymerase to initiate transcription as a portion of DNA. Generally, the promoter is a site that is adjacent to the target gene and located upstream of the target gene, and bound with RNA polymerase or a transcription factor which is a protein inducing RNA polymerase and may induce the enzyme or protein to be located at a correct transcription starting site. That is, the promoter has a specific gene sequence that is located at a 5' site of a gene to be transcribed in a sense strand so that RNA polymerase binds to the corresponding position directly or through a transcription factor to initiate mRNA synthesis for the target gene.

The promoter according to the present disclosure is desirably a cytomegalovirus (CMV) promoter including a sequence represented by SEQ ID NO: 1 from the viewpoint of increasing the gene expression.

As used herein, the term "ribozyme" is also called an RNA enzyme or catalytic RNA as a molecule consisting of an RNA molecule that acts like an enzyme or a protein containing the RNA molecule. As an RNA molecule with a clear tertiary structure, the ribozyme performs chemical reactions and has catalytic or autocatalytic properties. Some ribozymes cleave self- or other RNA molecules to inhibit the activity, and other ribozymes are known to catalyze the activity of aminotransferase of ribosomes. These ribozymes may include a hammerhead ribozyme, a VS ribozyme, a hairpin ribozyme, and the like.

The ribozyme according to the present disclosure may not only exhibit a selective anticancer effect by inhibiting the activity of a cancer-specific gene through the trans-splicing reaction of Group I introns, but also may be expressed in a form conjugated with an anti-cancer therapeutic gene to activate the anti-cancer therapeutic gene. Therefore, any type of ribozyme may be used as long as the ribozyme exhibits characteristics capable of inactivating the cancer-specific gene and activating the anti-cancer therapeutic gene.

The ribozyme according to the present disclosure may be desirably a ribozyme targeting hTERT mRNA as described above, and may serve to specifically cleave hTERT mRNA by targeting cancer cells in which hTERT is overexpressed to inhibit the expression and specifically express the therapeutic gene.

As used herein, the term "trans-splicing" means linking RNAs from different genes to each other. Desirably, an hTERT targeting trans-splicing group I ribozyme verified for trans-splicing ability by recognizing mRNA of cancer-specific hTERT may be used.

As used herein, the term "target gene" refers to a gene which is linked to mRNA of a cancer-specific gene by the ribozyme to induce the expression.

The target gene according to the present disclosure may be desirably an anti-cancer therapeutic gene or a reporter gene, and most desirably an anti-cancer therapeutic gene.

As used herein, the term "anti-cancer therapeutic gene" refers to a polynucleotide sequence encoding a polypeptide that exhibits a therapeutic effect when expressed in cancer cells. The anti-cancer therapeutic gene may be expressed in a conjugated form with the ribozyme or independently to exhibit anticancer activity. These anti-cancer therapeutic genes may be desirably one or more selected from the group consisting of a drug-sensitizing gene, a proapoptotic gene, a cytostatic gene, a cytotoxic gene, a tumor suppressor gene, an antigenic gene, a cytokine gene, and an anti-angiogenic gene, and most desirably a drug-sensitizing gene.

In the present disclosure, the anti-cancer therapeutic gene may be used alone or in combination with two or more genes.

The drug-sensitizing gene according to the present disclosure is a gene encoding an enzyme that converts a non-toxic prodrug into a toxic substance, and is also called a suicide gene because cells introduced with the gene are killed. That is, when the non-toxic prodrug is systemically administered to normal cells, the prodrug is converted into a toxic metabolite only in cancer cells to change the sensitivity to the drug, thereby destroying cancer cells. The drug-sensitizing gene may be desirably a Herpes simplex virus-thymidine kinase (HSVtk) gene using ganciclovir as a prodrug, or a cytosine deaminase (CD) gene of *E. coil* using 5-fluorocytosine (5-FC) as a prodrug, and most desirably an HSVtk gene including a sequence represented by SEQ ID NO: 4.

The proapoptotic gene according to the present disclosure refers to a nucleotide sequence that induces programmed apoptosis when expressed. The proapoptotic genes known to those skilled in the art may include p53, adenovirus E3-11.6K (derived from Ad2 and Ad5) or adenovirus E3-10.5K (derived from Ad), and adenovirus E4 genes, a p53 pathway gene and a gene encoding caspase.

The cytostatic gene according to the present disclosure refers to a nucleotide sequence that is expressed in cells to stop a cell cycle during the cell cycle. Examples thereof include p21, a retinoblastoma gene, an E2F-Rb fusion protein gene, genes (e.g., p16, p15, p18 and p19) encoding a cyclin-dependent kinase inhibitor, a growth arrest specific homeobox (GAX) gene, etc., but are not limited thereto.

The cytotoxic gene according to the present disclosure refers to a nucleotide sequence that is expressed in cells to exhibit a toxic effect. Examples thereof include nucleotide sequences encoding Pseudomonas exotoxin, ricin toxin, diphtheria toxin, and the like, but are not limited thereto.

The tumor suppressor gene according to the present disclosure refers to a nucleotide sequence that is expressed in a target cell to suppress a tumor phenotype or induce apoptosis. Representatively, the tumor suppressor gene may include tumor necrosisfactor-α (TNF-α), p53 gene, APC gene, DPC-4/Smad4 gene, BRCA-1 gene, BRCA-2 gene, WT-1 gene, retinoblastoma gene, MMAC-1 gene, adenomatous polyposis coil protein, deleted colon cancer (DCC) gene, MMSC-2 gene, NF-1 gene, nasopharyngeal cancer suppressor gene located on chromosome 3p21.3, MTS1 gene, CDK4 gene, NF-1 gene, NF-2 gene, VHL gene or programmed death-1 (sPD-1).

The antigenic gene according to the present disclosure refers to a nucleotide sequence that is expressed in a target cell to produce a cell-surface antigenic protein that may be recognized by an immune system. Examples of the antigenic gene known to those skilled in the art may include carcinoembryonic antigen (CEA) and p53.

The cytokine gene according to the present disclosure refers to a nucleotide sequence that is expressed in a cell to produce cytokines. Representatively, the cytokine gene may include GMCSF, interleukins IL-1, IL-2, IL-4, IL-12, IL-10, IL-19, and IL-20, interferons α, β, and γ (interferon α-2b), fusions such as interferon α-2α-1, etc.

The anti-angiogenic gene according to the present disclosure refers to a nucleotide sequence that is expressed to release an anti-angiogenic factor out of the cell. Examples thereof may include angiostatin, vascular endothelial growth factor (VEGF) inhibitor, endostatin, and the like.

As used herein, the term "herpes simplex virus-thymidine kinase (HSVtk)" refers to a thymidine kinase derived from a herpes simplex virus. This enzyme is a representative example of a drug-sensitizing gene that converts a non-toxic prodrug into a toxic substance to kill the cells transfected with the gene. In the present disclosure, the HSVtk gene may be used as an anti-cancer therapeutic gene that is expressed in a form conjugated to the ribozyme according to the present disclosure to exhibit anticancer activity. These HSVtk genes may be desirably disclosed in Genbank registration Nos. AAP13943, P03176, AAA45811, P04407, Q9QNF7, KIBET3, P17402, P06478, P06479, AAB30917, P08333, BAB84107, AAP13885, AAL73990, AAG40842, BAB11942, NP_044624, NP_044492, CAB06747, and the like.

As used herein, the term "reporter gene" is a gene used to monitor the introduction of a recombinant vector or the expression efficiency of a ribozyme according to an embodiment of the present disclosure, and may be used with any gene that may be monitored without damage to infected cells or tissues without limitation. Desirably, the reporter gene may be luciferase, green fluorescent protein (GFP), modified green fluorescent protein (mGFP), enhanced green fluorescent protein (EGFP), red fluorescent protein (RFP), modified red fluorescent protein (mRFP), enhanced red fluorescent protein (ERFP), blue fluorescent protein (BFP), enhanced blue fluorescent protein (EBFP), yellow fluorescent protein (YFP), enhanced yellow fluorescence protein (EYFP), cyan fluorescent protein (CFP) or enhanced cyan fluorescent protein (ECFP).

The expression level of the cancer cell-specific ribozyme may be observed by inserting the reporter gene into the target gene, and particularly, the ribozyme of the present disclosure includes a promoter and a microRNA target site, which is not expressed in normal cells, but may be specifically expressed in cancer cells. It is obvious for those skilled in the art to be applied to diagnose whether cancer has occurred in a specific tissue using the ribozyme.

### [Gene delivery system]

Another aspect of the present disclosure is a gene delivery system including a recombinant vector according to the present disclosure.

As used herein, the term "gene delivery system" refers to a system capable of increasing expression efficiency by increasing the delivery efficiency of a target gene and/or nucleic acid sequence into a cell, and may be classified into a virus-mediated system and a non-viral system.

The virus-mediated system is known to have relatively higher intracellular gene delivery efficiency than that of the non-viral system by using viral vectors such as a retroviral vector and an adenovirus vector and using a unique intracellular penetration mechanism of viruses that infect human cells. In addition, the non-viral vector introduced into the cell has a problem in that an endosome is fused with a lysosome and then genes are degraded in the endolysosome, but the viral vector has an advantage of having high gene delivery efficiency due to low gene loss caused by a mechanism for delivering the gene into the nucleus without passing through the lysosome.

The viral vector that may be used in the present disclosure may be vectors derived from retrovirus, adenovirus, adeno-associated virus, and the like, as described above in the recombinant vector. These viral vectors may be assembled into viral particles and then introduced into cells by a transduction method such as infection.

In an embodiment of the present disclosure, a recombinant adenovirus including the above-described recombinant vector was fabricated as an example of a gene carrier. That is, the recombinant adenovirus performs a function of delivering a recombinant vector expressing a trans-splicing ribozyme specific for a cancer-specific gene into a target cell (e.g., cancer cell), and the recombinant vector delivered into the cell is expressed by an intracellular transcription system. The expressed trans-splicing ribozyme may insert a target gene linked to the ribozyme into a transcript of the cancer-specific gene that is rich in cancer cells. In this specification, adenovirus containing a recombinant vector ECRT-122T expressing the trans-splicing ribozyme of the present disclosure is named RZ-001.

The non-viral system is a method using a cationic lipid carrier, a cationic polymer carrier or the like as a delivery carrier for a nucleic acid and/or gene, or using an electroporation method.

The cationic lipid carrier is a method of forming a complex with a negatively charged gene and an expression vector or nucleic acid containing the gene using a positive charge of nanometer-sized liposomes or lipid nanoparticles mainly consisting of cationic lipids and then delivering the complex into cells by phagocytosis. The complex delivered into the cell is first delivered from the endosome to the lysosome and then released into the cytoplasm to be expressed. The cationic polymer carrier delivers a gene in a similar manner to a cationic lipid carrier, except for using a polymer instead of lipids, and a representative cationic polymer includes polyethyleneimine, poly-L-lysine, chitosan, and the like.

Accordingly, a complex formed by combining the recombinant vector of the present disclosure with the cationic lipid carrier or the cationic polymer carrier may be used as a gene carrier.

In the present disclosure, the gene delivery system includes the above-described recombinant vector, and both a virus-mediated system and a non-viral system may be used, but it is desirable to use a virus-mediated system.

### [Ribozyme]

Another aspect of the present disclosure is a ribozyme expressed from the recombinant vector according to the present disclosure.

Details on the recombinant vector or ribozyme according to the present disclosure are as described above.

### [Pharmaceutical composition]

Another aspect of the present disclosure is a pharmaceutical composition for preventing or treating cancer including a recombinant vector, a gene delivery system including the recombinant vector, or a ribozyme according to the present disclosure as an active ingredient.

The pharmaceutical composition may be combined with radiotherapy and may be used to enhance the efficacy of radiotherapy.

Details on the recombinant vector, the gene delivery system, or the ribozyme according to the present disclosure are as described above.

As used herein, the term "cancer" means a condition in which there is a problem in a regulatory function of normal division, differentiation, and apoptosis of cells, and thus the cells are abnormally excessively proliferated and invade surrounding tissues and organs to form a lump and destroy or transform an existing structure.

Non-limiting examples of cancer according to the present disclosure may include liver cancer, thyroid cancer, testicular cancer, bone cancer, glioblastoma, ovarian cancer, brain cancer, gallbladder cancer, biliary tract cancer, colon cancer, head and neck cancer, lymphoma, bladder cancer, leukemia, peritoneal cancer, small intestine cancer, esophageal cancer, renal pelvis cancer, kidney cancer, heart cancer, duodenal cancer, eye cancer, urethral cancer, breast cancer, stomach cancer, prostate cancer, uterine cancer, lung cancer, spinal tumor, pancreatic cancer, and melanoma, desirably liver cancer, glioblastoma, and biliary tract cancer, and more desirably liver cancer.

The cancer according to the present disclosure may be cancer resistant to radiotherapy.

In addition, in the cancer according to the present disclosure, desirably, the copy number (expression level) of miR-122 expressed in cancer tissues may be 100 times less than the copy number of the ribozyme expressed in cancer tissues by the pharmaceutical composition.

In an embodiment of the present disclosure, as a result of comparing the expression level of the hTERT targeting ribozyme with miR-122T capable of inducing apoptosis with the expression level of miR-122 in cells, as the ratio of miR-122 to the ribozyme was increased, the expression of the ribozyme was reduced and the apoptosis inducing effect was reduced. Accordingly, the injection amount of adenovirus expressing the ribozyme may be determined by inferring the amount of the ribozyme to exhibit the anticancer effect according to the expression level of miR-122 in cancer tissues. Specifically, if the minimum copy number of miR-122 is about 100 times or more the ribozyme copy number, the function (expression) of the ribozyme having an miR-122 target site is attenuated, and thus it was confirmed that high anticancer efficacy may be obtained if the copy number of miR-122 expressed in cancer tissues is 100 times less than the copy number of the ribozyme expressed in cancer tissues by the pharmaceutical composition according to the present disclosure.

In addition, the cancer according to the present disclosure may be desirably cancer in which miR-122 is not substantially expressed in cancer tissues. The "cancer in which miR-122 is not substantially expressed in cancer tissues" means cancer in which miR-122 is expressed in cancer tissues, but the copy number of miR-122 expressed in cancer tissues is low enough not to substantially affect the function of the ribozyme having the miR-122 target site.

In an embodiment of the present disclosure, the anti-cancer efficacy of the ribozyme according to the present disclosure was confirmed in colon cancer, glioblastoma, melanoma, cervical cancer, lung cancer, osteosarcoma, breast cancer, and biliary tract cancer cell lines in which miR-122 is not substantially expressed in cancer tissues.

In addition, liver cancer according to the present disclosure may be desirably caused by at least one selected from the group consisting of hepatitis B virus, hepatitis C virus which reduces the expression of miR-122 in liver cancer tissue, alcohol, chronic hepatitis, cirrhosis, non-alcoholic fatty liver disease, aflatoxin, and family history.

In addition, the present disclosure may provide the recombinant vector, the gene delivery system, and the ribozyme described above as a pharmaceutical composition combined with radiotherapy. The pharmaceutical composition of the present disclosure is combined with radiotherapy to effectively reduce the size of tumor tissue even at a low dose of the pharmaceutical composition and a low dose of radiation. Particularly, the pharmaceutical composition of the present disclosure is administered after radiotherapy to primarily reduce the size of the tumor tissue by radiation, increase tissue penetration of the drug, and induce the death of cancer cells resistant to radiation by the pharmaceutical composition of the present disclosure, thereby enabling effective cancer therapy.

Therefore, the pharmaceutical composition of the present disclosure is combined with radiotherapy and may be administered simultaneously or sequentially with radiotherapy. Desirably, the pharmaceutical composition of the present disclosure may be administered after performing the radiotherapy, and if the radiotherapy is preceded, in subsequent treatment, the pharmaceutical composition of the present disclosure may be administered simultaneously with radiotherapy. In addition, the pharmaceutical composition of the present disclosure may be provided for the treatment of radiation-resistant cancer.

In the present disclosure, 'simultaneously' means that the radiotherapy and the administration of the pharmaceutical composition of the present disclosure are performed within 24 hours, and 'sequentially' means that the radiotherapy and the administration of the pharmaceutical composition are performed over 24 hours.

As used herein, the term "prevention" refers to all actions that inhibit the proliferation, metastasis, and acquisition of aggression of cancer cells or delay the onset of cancer by administering the pharmaceutical composition according to the present disclosure.

As used herein, the term "treatment" refers to all actions that improve cancer or beneficially change its symptoms by administering the composition including the vector according to the present disclosure.

The pharmaceutical composition according to the present disclosure may further include a pharmaceutically acceptable carrier, an excipient, or a diluent. Examples of the pharmaceutically acceptable carrier, the excipient, and the diluent that may be used in the pharmaceutical composition of the present disclosure may include lactose, dextrose, sucrose, sorbitol, mannitol, xylitol, erythritol, maltitol, starch, acacia gum, alginate, gelatin, calcium phosphate, calcium silicate, calcium carbonate, cellulose, methylcellulose, polyvinylpyrrolidone, water, methylhydroxybenzoate, propylhydroxybenzoate, talc, magnesium stearate, mineral oils, and the like.

The pharmaceutical composition of the present disclosure may be administered orally or parenterally depending on a desired method, but is desirably administered parenterally.

According to an embodiment of the present disclosure, the pharmaceutical composition according to the present disclosure may be directly administered intravenously, intraarterially, subcutaneously, or into cancer tissue, or administered as injections. The injection according to the present disclosure may be a form dispersed in a sterile medium so as to be used as it is when administered to a patient, or may also be administered after dispersing in an appropriate concentration by adding distilled water for injection during administration. In addition, when prepared as the injection, the pharmaceutical composition may be mixed with buffers, preservatives, analgesics, solubilizers, tonicity agents, stabilizers, etc., and may be prepared in unit dosage ampoules or multiple dosage forms.

A dose of the pharmaceutical composition of the present disclosure varies according to the condition and body weight of a patient, the degree of a disease, a drug form, and the route and period of administration, but may be properly selected by those skilled in the art. Meanwhile, the pharmaceutical composition according to the present disclosure may be used alone or in combination with auxiliary therapy methods such as surgical therapy.

Hereinafter, the present specification will be described in detail with reference to Examples for a specific description. However, Examples according to the present specification may be modified in various different forms, and it is not interpreted that the scope of the present specification is limited to Examples to be described in detail below. Examples of the present specification will be provided for more completely explaining the present specification to those skilled in the art.

### Example: Construction of hTERT targeting trans-splicing ribozyme recombinant vector

In order to construct an hTERT targeting trans-splicing ribozyme having an miR-122 target site miR-122T and exhibiting high expression efficiency, a recombinant vector was designed by modifying a conventional trans-splicing ribozyme.

Specifically, a CMV promoter was introduced into a conventional trans-splicing ribozyme that targeted a +21 site of hTERT mRNA, had a 326-nt long antisense sequence (SEQ ID NO: 8), and had HSV-tk as a therapeutic gene. An SV40 intron splicing donor/acceptor (SD/SA) sequence was inserted between the CMV promoter and the ribozyme to increase transcription efficiency. A posttranscriptional regulatory element of Woodchuck Hepatitis Virus, WPRE, was inserted into the 3' site of the therapeutic gene HSV-tk, to increase protein expression efficiency of the therapeutic gene, and three copies of miR-122T were inserted into the 3' end site of a construct so as to be regulated by miR-122.

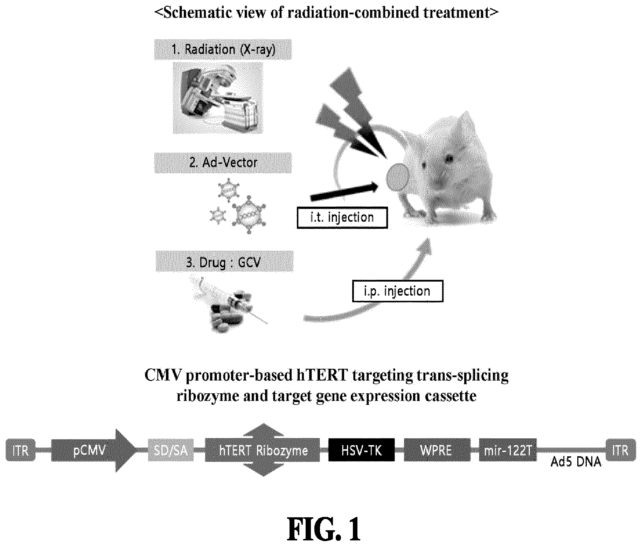

The overall vector structure of the designed trans-splicing ribozyme was shown in FIG. 1, and was named ECRT-122T.

### Experimental Example 1. Animal experiment for confirming anti-cancer efficacy of ECRT-122T (in vivo)

Tumor formation was induced by subcutaneously injecting 6-week-old male Balb/c-nunu mice with 1 × 10⁷ (100 µl) of an LN229 cell line or 5 × 10⁶ (100 µl) of a U87MG cell line as glioblastoma cells. When the tumor grew to a certain size, adenovirus expressing ECRT-122T was injected at a dose of 1.0 × 10⁹ VP (100 µl) a total of 3 times, once every 2 days. GCV was injected intraperitoneally (I.P. injection) at a dose of 50 mg/kg twice a day for 10 days from 24 hours after the first virus injection (20 times in total).

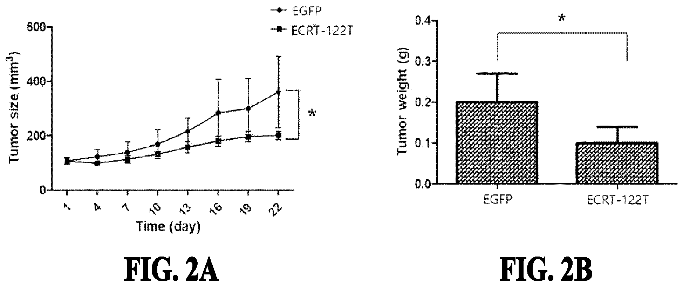

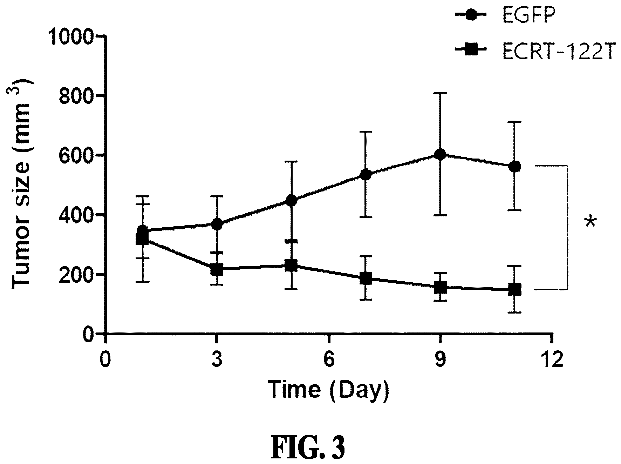

As a result of the experiment, as shown in FIG. 2, it was found that the tumor size continued to increase in a control group (Ad-EGFP injection), but in an experimental group injected with ECRT-122T, the tumor hardly grew, and the tumor weight was also lower in the experimental group injected with ECRT-122T. In addition, as shown in FIG. 3, the tumor growth inhibitory effect of ECRT-122T was confirmed even in the experiment using the U87MG cell line.

From the result of Experimental Example, it was confirmed that the ribozyme expressed from ECRT-122T may be effectively applied to various carcinomas that did not express miR-122 in addition to liver cancer. In addition, when ECRT-122T was administered systemically or locally as an anticancer agent for cancers other than liver cancer, the ECRT-122T may be introduced into the normal liver, and in this case, the toxicity induction in the normal liver may be suppressed through the action of miR-122T.

### Experimental Example 2: Confirmation of toxicity of ECRT-122T according to GCV treatment

### 2-1. Untreated GCV

ECRT-122T adenovirus was injected once into normal ICR mice, and AST and ALT levels were measured at 15 and 29 days after injection.

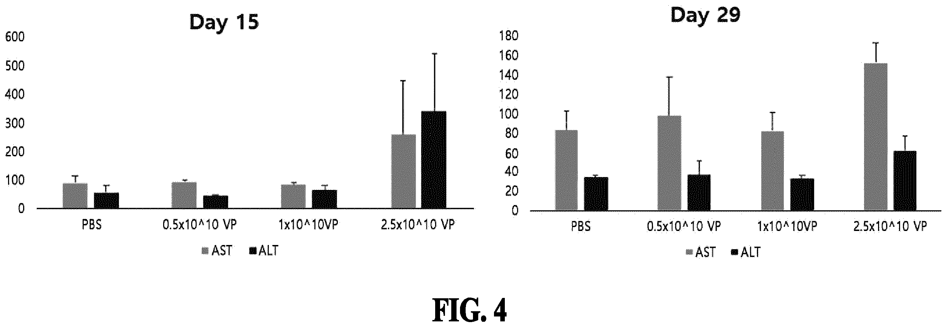

As a result, as shown in FIG. 4, it was confirmed that slight toxicity was shown in a 2.5 × 10¹⁰ VP experimental group, and in a remaining-dose experimental group, AST and ALT levels were similar to those of a PBS injection group to have almost no toxicity.

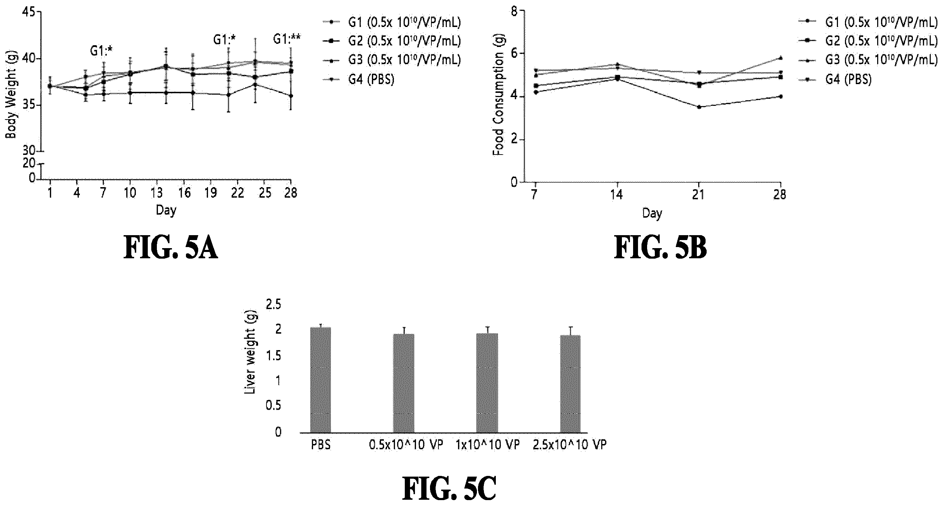

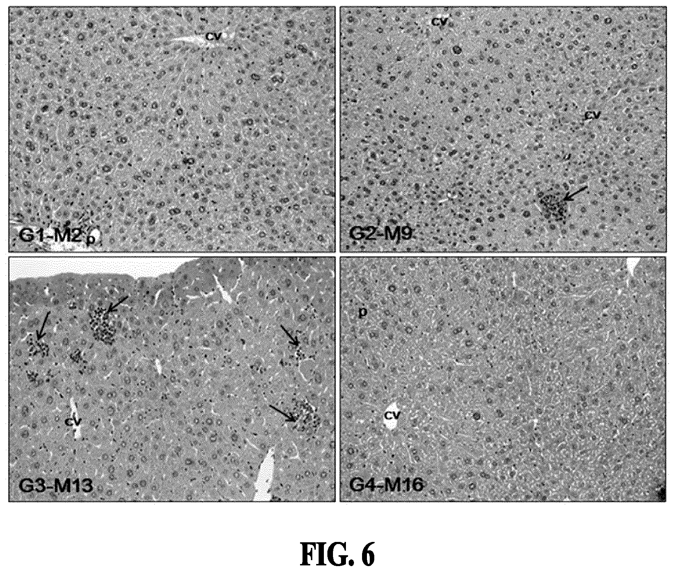

In addition, as shown in FIG. 5, no abnormal findings were confirmed in the body weight, feed consumption, and liver weight in mice during the experiment period. As a result of a histopathological test of the liver conducted after 29 days after a single intravenous administration of adenovirus, as shown in FIG. 6, it could be seen that hepatic necrosis (arrow) and inflammation were observed in a 2.5 × 10¹⁰ VP experimental group (G3) to induce mild liver damage. In a 1.0 × 10¹⁰ VP experimental group (G2), it was confirmed that inflammatory cells were locally infiltrated (arrow), but liver damage was not induced. In FIG. 6, cv means a central vein, and p means a portal area.

The histopathological test results were summarized in Table 1 below.

**[Table 1]**

| Histopathology/Groups | G1 | G2 | G3 | G4 |
|---|---|---|---|---|
| | 0.5 × 10¹⁰ VP | 1.0 × 10¹⁰ VP | 2.5 × 10¹⁰ VP | Vehicle (PBS) |
| No. examined | 4 | 5 | 5 | 5 |
| No specific lesion | 4 (100) | 4 (80) | 0 (0.00) | 5 (100) |
| Inflammatory foci with hepatocyte necrosis | 0 (0.00) | 1 (20.0) | 4 (80.0) | 0 (0.00) |
| *Grades: Minimal* | 0 | 1 | 3 | 0 |
| *Mild* | 0 | 0 | 1 | 0 |
| Cell infiltration, mononuclear cells, periductal, focal | 0 (0.00) | 0 (0.00) | 2 (40.0) | 0 (0.00) |
| *Grades: Minimal* | 0 | 0 | 2 | 0 |

### 2-2. Treated GCV

ECRT-122T adenovirus was injected into normal ICR mice, GCV was administered intraperitoneally twice a day for 10 days, and then AST and ALT levels were measured.

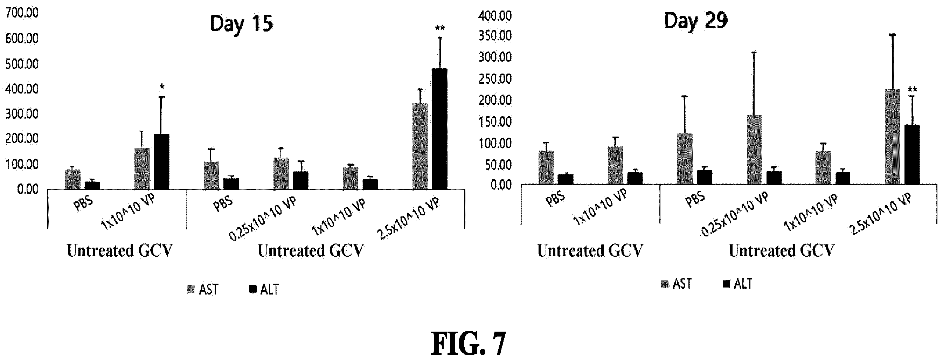

As a result, as shown in FIG. 7, it was confirmed that in the remaining-dose experimental group except for the 2.5 × 10¹⁰ VP experimental group, AST and ALT levels were similar to those of a control group (PBS injection group) to have almost no toxicity.

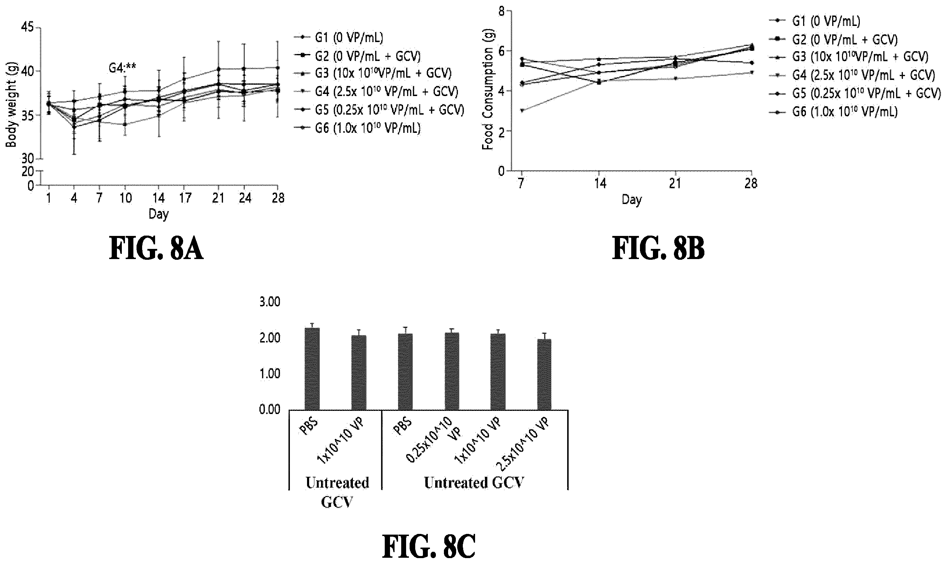

In addition, as shown in FIGS. 8(a) and 8(b), there was no significant change in the body weight and feed consumption of the mice during the experiment, and as shown in FIG. 8(c), no abnormal findings were found even in the liver weight.

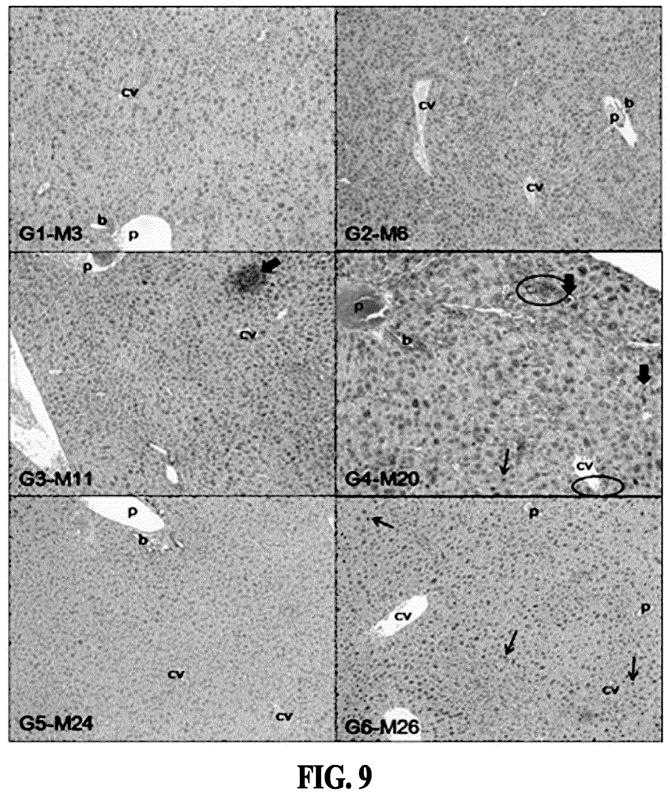

As a result of histopathological analysis of the liver, as shown in FIG. 9, no abnormal findings were found in a PBS injected group (G1), a PBS + GCV administered group (G2), and a 0.25 × 10¹⁰ VP + GCV administered group (G5). On the other hand, in a 1.0 × 10¹⁰ VP + GCV administered group (G3), a local microabscess (bold arrow) formed by neutrophil infiltration was confirmed, but liver damage was not induced. In a 2.5 × 10¹⁰ VP + GCV administered group (G4), it was confirmed that hypertrophied hepatocytes with large nuclei, necrotic hepatocytes (bold arrows), multiple inflammatory cell infiltration (circles), increased hepatocyte mitosis (bold arrows), and lymphocytic cell infiltration around bile tubules (b) were observed to induce the liver damage. In G6 (1.0 × 10¹⁰ VP injected group) that was not administered with GCV, the number of mitotic hepatocytes (arrow) was slightly increased.

The histopathological test results were summarized in Table 2 below.

**[Table 2]**

| Histopathology/ Groups | G1 | G2 | G3 | G4 | G5 | G6 |
|---|---|---|---|---|---|---|
| | Vehicle (PBS) | PBS + GCV | 1.0 × 10¹⁰ VP + GCV | 2.5 × 10¹⁰ VP + GCV | 0.25 × 10¹⁰ VP + GCV | 1.0 × 10¹⁰ VP |
| No. examined | 5 | 5 | 5 | 5 | 5 | 5 |
| No specific lesion | 5 (100) | 4 (80.0) | 3 (60.0) | 0 (0.00) | 5 (100) | 3 (60.0) |
| Cell infiltration, mononuclear or mixed cells, multifocal | 0 (0.00) | 1 (20.0) | 1 (20.0) | 4 (80.0) | 0 (0.00) | 0 (0.00) |
| *Grades: Minimal* | 0 | 1 | 1 | 0 | 0 | 0 |
| *Mild* | 0 | 0 | 0 | 4 | 0 | 0 |
| Microabcess, focal | 0 (0.00) | 0 (0.00) | 1 (20.0) | 0 (0.00) | 0 (0.00) | 0 (0.00) |
| *Grades: Minimal* | 0 | 0 | 1 | 0 | 0 | 0 |
| Hepatocytomegaly, diffuse | 0 (0.00) | 0 (0.00) | 0 (0.00) | 5 (100) | 0 (0.00) | 0 (0.00) |
| *Grades: Minimal* | 0 | 0 | 0 | 1 | 0 | 0 |
| *Mild* | 0 | 0 | 0 | 2 | 0 | 0 |
| *Moderate* | 0 | 0 | 0 | 2 | 0 | 0 |
| Necrotic hepatocytes | 0 (0.00) | 0 (0.00) | 0 (0.00) | 3 (60.0) | 0 (0.00) | 0 (0.00) |
| *Grades: Minimal* | 0 | 0 | 0 | 2 | 0 | 0 |
| *Mild* | 0 | 0 | 0 | 1 | 0 | 0 |
| Hepatocytic mitosis, increased, diffuse | 0 (0.00) | 0 (0.00) | 0 (0.00) | 4 (80.0) | 0 (0.00) | 1 (20.0) |
| *Grades: Minimal* | 0 | 0 | 0 | 2 | 0 | 1 |
| *Mild* | 0 | 0 | 0 | 1 | 0 | 0 |
| *Moderate* | 0 | 0 | 0 | 1 | 0 | 0 |
| Oval cell hyperplasia | 0 (0.00) | 0 (0.00) | 0 (0.00) | 3 (60.0) | 0 (0.00) | 0 (0.00) |
| *Grades: Minimal* | 0 | 0 | 0 | 3 | 0 | 0 |
| Pericholangitis, (multi)focal | 0 (0.00) | 0 (0.00) | 0 (0.00) | 3 (60.0) | 0 (0.00) | 1 (20.0) |
| *Grades: Minimal* | 0 | 0 | 0 | 3 | 0 | 1 |

AST and ALT levels and histopathological test results showed that when adenovirus was administered intravenously at a dose of 2.5 × 10¹⁰ VP/head and GCV was administered twice a day for 10 days, liver damage including hepatocyte necrosis and inflammation was induced. However, when administered alone or in combination with GCV at 0.25 × 10¹⁰ VP/head and 1.0 × 10¹⁰ VP/head, AST and ALT levels related to liver damage were increased up to 15 days after administration, but in a histological test performed on day 29, there was observed no significant toxicological changes in the liver that were determined to be related to adenovirus administration.

### Comparative Example 1: Comparison of anticancer efficacies of CRT-122T and ECRT-122T

Tumor formation was induced by injecting SNU398 cells into a mouse xenograft subcutaneous model, and when the tumor grew above over a certain size, adenovirus containing a CRT-122T or ECRT-122T vector was intratumorally injected (I.T. injection) into the cancer tissue at a dose of 1 × 10⁹ VP, once every two days, twice in total, and GCV was intraperitoneally administered (I.P. injection) at a dose of 50 mg/kg twice a day for 10 days from 24 hours after the first adenovirus injection (20 times in total). After adenovirus injection, the mice were raised and the tumor size and body weight were measured every 3 days, and after 22 days, the mice were sacrificed and the final tumor size, liver weight, and aspartate transaminase (AST), and alanine transaminase (ALT) levels were measured.

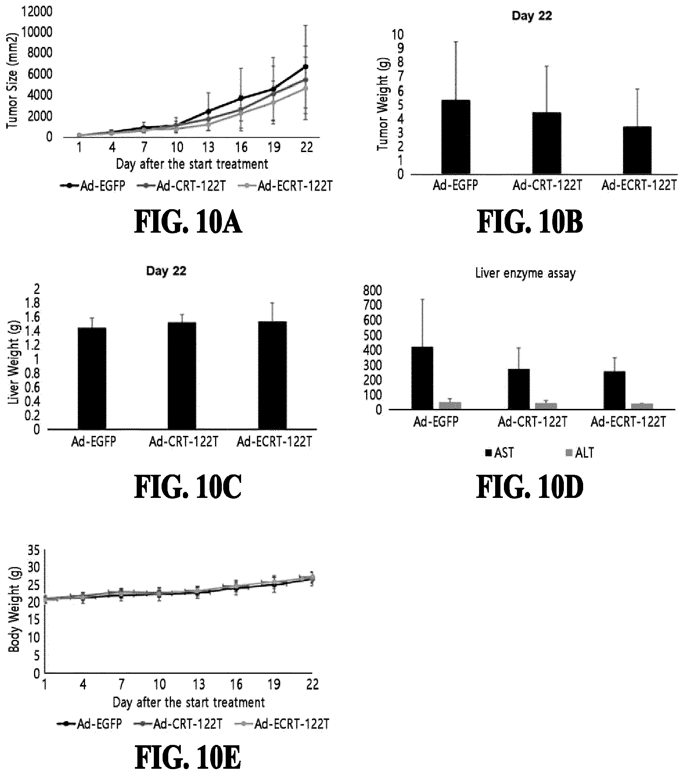

As a result, as shown in FIGS. 10(a) and 10(b), it was confirmed that the anticancer efficacy of ECRT-122T was superior to that of CRT-122T. There was no significant difference in the body weight and liver weight of mice between the experimental groups, and AST and ALT levels showed that adenovirus did not induce liver toxicity (FIGS. 10(c) to 10(e)).

Through the results, it was confirmed that a sufficient anticancer effect could be obtained even by injecting only a small amount of adenovirus, and the anticancer efficacy of ECRT-122T was significantly superior to CRT-122T.

### Comparative Example 2. Comparison of efficacy according to radiation-combined treatment

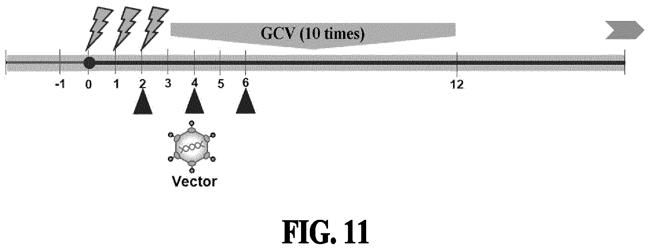

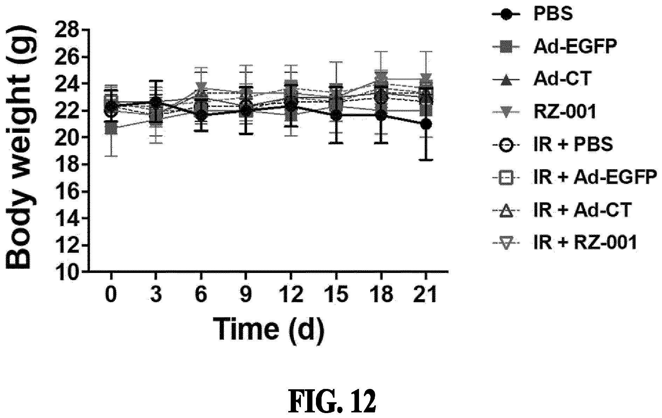

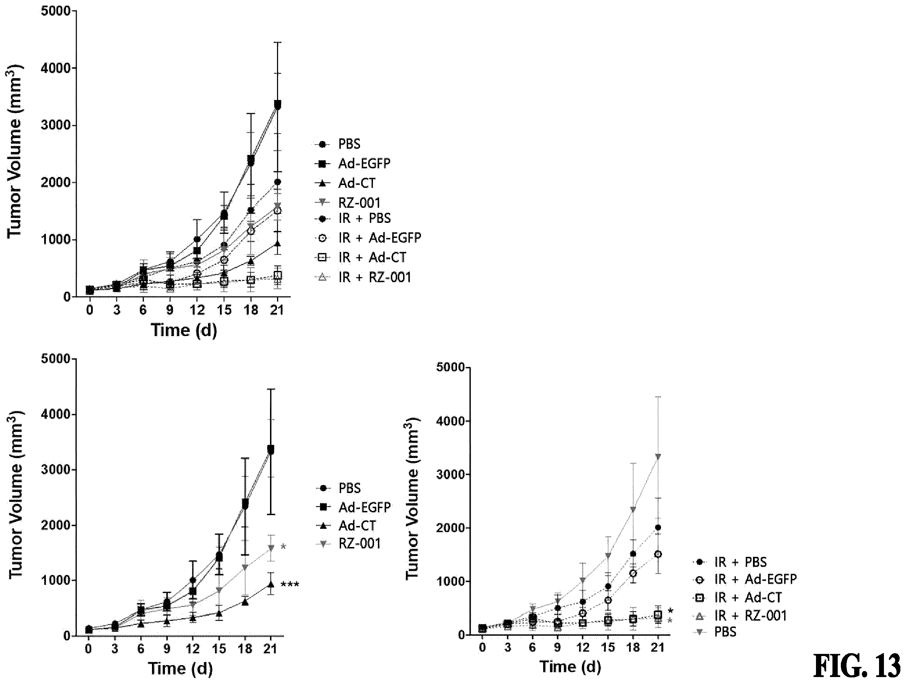

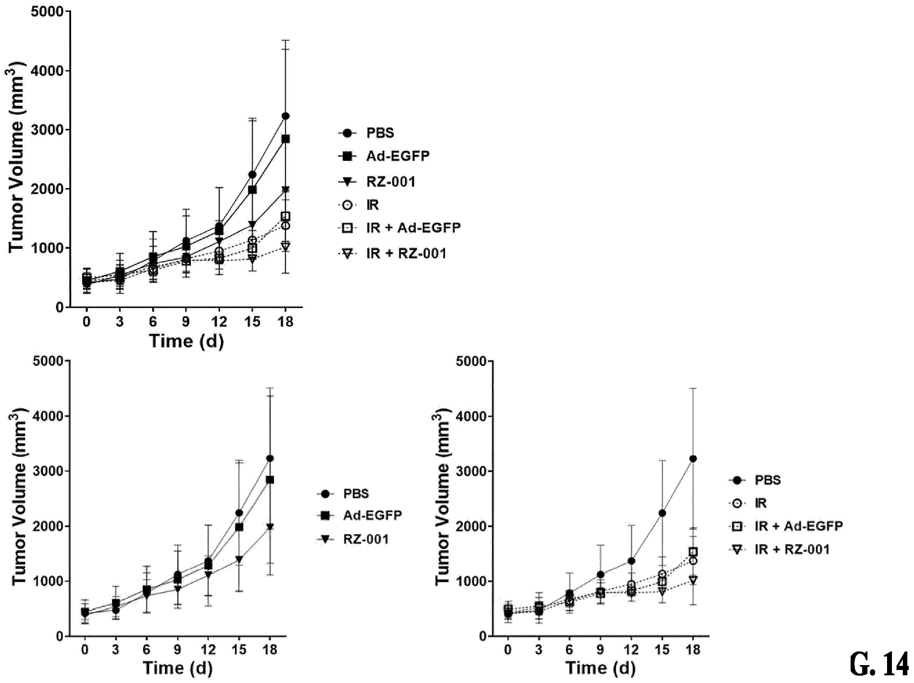

In order to confirm an anticancer effect according to a combination of adenovirus and radiotherapy, when tumor formation was induced by injecting SNU398 cells (FIG. 13) and Hep3B cells (FIG. 14) into a mouse xenograft subcutaneous model (6-7 week-old male BALB/C nude mice), respectively, and then the tumor reached 100 mm³, 2 gray doses of iridium (IR) were injected every day for 3 days, and then adenovirus was administered. On the last day of radiotherapy, after the last irradiation treatment, an adenovirus vector was administered once a day, three times at two-day intervals (one dose of adenovirus: 1 × 10⁹ VP). 24 hours after the first adenovirus treatment, GCV (ganciclovir) was administered intraperitoneally at 10 mg/kg daily for 10 days (see FIG. 11). Adenovirus administration was divided into RZ-001, Ad-EGFP, Ad-CT, and PBS groups, and the experiment was divided into a radiation and adenovirus combined treatment group (IR + Ad) and a radiation or adenovirus-alone treatment group, and the efficacies thereof were compared by measuring the body weight and tumor size of the mouse (FIGS. 12 to 14). As a result, the radiation and adenovirus combined treatment group was able to significantly reduce tumor growth compared to the untreated, radiation, or adenovirus-alone treatment group, and in particular, the smallest tumor size was confirmed in the IR + RZ-001 group.

### Comparative Example 3. Searching of optimal RZ-001 and radiation-combined therapy

An optimal treatment protocol was to be established to maximize the anticancer effect according to a combination of adenovirus and radiotherapy. When tumor formation was induced by injecting SNU398 cells into a mouse xenograft subcutaneous model (6-7 week-old male BALB/C nude mice), and the tumor reached 100 mm³, administration of adenovirus and irradiation of radiation were performed under various conditions. The radiation was administered at 2 Gy once, and adenovirus was administered at 1 × 10⁹ VP/head. The administration of adenovirus and irradiation of radiation in each group were conducted under the following conditions:
G1: PBS
G2: RZ-001 alone. Administered once
G3: RZ-001 alone. Administered a total of 3 times at 2-day intervals
G4: Radiation alone. Irradiation of 2 Gy once
G5: Radiation alone. Irradiation of 2 Gy, total 3 times at 1-day intervals
G6: Administration of RZ-001 once on the same day after irradiation of 2 Gy once
G7: Administration of RZ-001 total 3 times at 2-day intervals from the same day after irradiation of 2 Gy once
G8: Irradiation of 2 Gy, total 3 times at 1-day intervals. Administration of RZ-001 once on the same day after last irradiation
G9: Radiation of 2 Gy, total 3 times at 1-day intervals. Administration of RZ-001 total 3 times at 2-day intervals from the same day after last irradiation
50 mg/kg of GCV (ganciclovir) was intraperitoneally administered daily for 10 days, 24 hours after the last radiotherapy in the radiation-alone treatment group, and 24 hours after the first adenovirus treatment in the adenovirus-alone or combined treatment group (FIG. 14). Adenovirus administration was RZ-001, and the experiment was divided into the radiation and adenovirus combined treatment group (IR + RZ-001) and the radiation or adenovirus-alone treatment group, and the efficacies were compared by measuring the body weights and the tumor sizes of the mice.

The treatment of each group was shown in Table 3 below.

**[Table 3]**

| **Group** | **Treatment** | **Route of administration** | **Dose** | **Number of Dose** | **Dose volume** |
|---|---|---|---|---|---|
| G1 | PBS | i.t | - | 3 | 30 mL |
| | GCV | i.p | 50 mg/kg | 10 | 100 mL |
| G2 | RZ-001 | i.t | 1 × 10⁹ VP | 1 | 30 mL |
| | GCV | i.p | 50 mg/ kg | 10 | 100 mL |
| G3 | RZ-001 | i.t | 1 × 10⁹ VP | 3 | 30 mL |
| | GCV | i.p | 50 mg/ kg | 10 | 100 mL |
| G4 | IR | - | 2 Gy | 1 | - |
| | PBS | i.t | - | | 30 mL |
| | GCV | i.p | 50 mg/ kg | 10 | 100 mL |
| G5 | IR | - | 2 Gy | 3 | - |
| | PBS | i.t | - | | 30 mL |
| | GCV | i.p | 50 mg/ kg | 10 | 100 mL |
| G6 | IR | | 2 Gy | 1 | - |
| | RZ-001 | i.t | 1 × 10⁹ VP | 1 | 30 mL |
| | GCV | i.p | 50 mg/ kg | 10 | 100 mL |
| G7 | IR | | 2 Gy | 1 | - |
| | RZ-001 | i.t | 1 × 10⁹ VP | 3 | 30 mL |
| | GCV | i.p | 50 mg/ kg | 10 | 100 mL |
| G8 | IR | | 2 Gy | 3 | - |
| | RZ-001 | i.t | 1 × 10⁹ VP | 1 | 30 mL |
| | GCV | i.p | 50 mg/ kg | 10 | 100 mL |
| G9 | IR | | 2 Gy | 3 | - |
| | RZ-001 | i.t | 1 × 10⁹ VP | 3 | 30 mL |
| | GCV | i.p | 50 mg/ kg | 10 | 100 mL |

As a result, the radiation and adenovirus combined treatment group was able to significantly reduce the tumor growth compared to untreated, the radiation, or adenovirus-alone treatment group.

Specifically, tumor growth inhibition was confirmed at 43.6% in the once administration group (G2) and 58.6% in the three-time administration group (G3) in the RZ-001 administration group compared to the PBS administration group (G1). In the radiation-alone treatment group, tumor growth inhibition was confirmed at 36.4% in the 2 Gy-once irradiation group (G4) and 69.9% in the 2 Gy-three-time irradiation group (G5) compared to the PBS administration group. In addition, in the combined treatment group, compared to the PBS administration group (G1), tumor growth inhibition was confirmed at 56.9% in a radiation 2 Gy-once + RZ-001-once administration group (G6), 70% in a radiation 2 Gy-once + RZ-001-three-time administration group (G7), 75.6% in a radiation 2 Gy-three-time + RZ-001-once administration group (G8), and 86.1% in a radiation 2 Gy-three-time + RZ-001-three-time administration group (G9).

In summary, a higher anticancer effect may be obtained with three-time irradiation of 2Gy radiation compared to once irradiation, and high anticancer efficacy was exhibited under the condition of three-time administration compared to once administration of RZ-001. In addition, a higher anticancer effect was confirmed in the radiation 2 Gy-three-time + RZ-001-once group (G8) than the RZ-001-alone-three-time administration group (G3) and the radiation 2 Gy-once + RZ-001-three-time administration group (G7), and as a result, it was found that only once administration of RZ-001 exhibited a synergistic effect in the therapy method combined with radiotherapy.

### Comparative Example 4. Confirmation of intracellular signaling changes according to treatment with RZ-001 alone and combination with irradiation

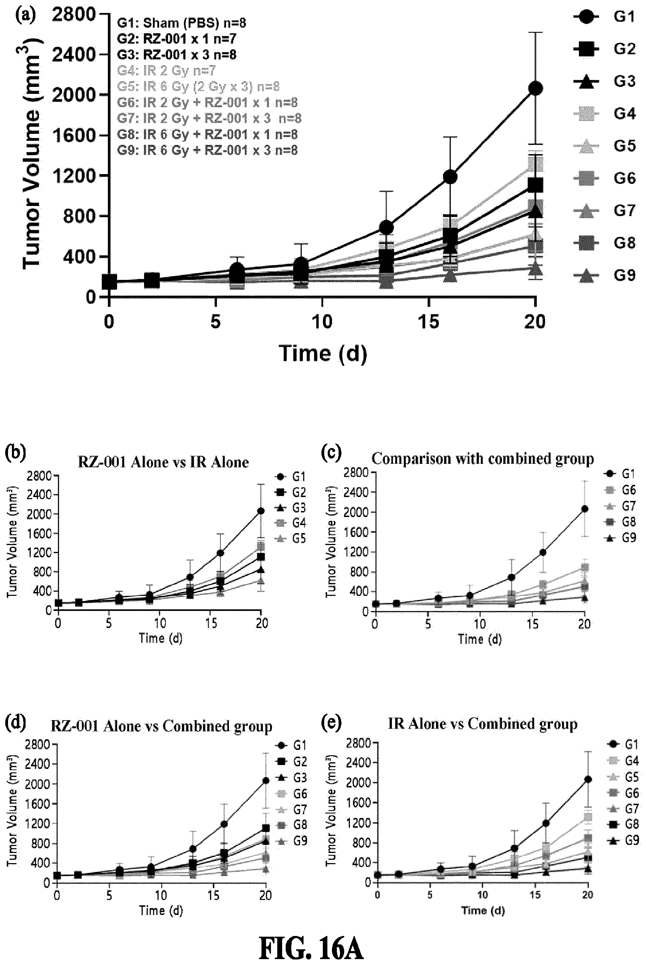

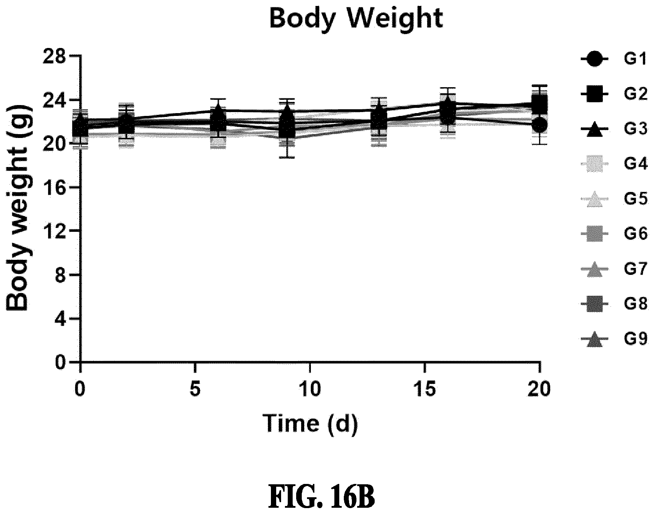

Changes in intracellular signaling were confirmed after irradiation and/or RZ-001 infection in a Hep3B human liver cancer cell line. To this end, in the adenovirus-alone treatment group, 40 moi of adenovirus was infected in Hep3B cells under untreated GCV, and in the irradiation combined treatment group, 40 moi of adenovirus was infected after irradiation of 4 Gy, and then intracellular signaling changes were confirmed over time under untreated GCV. That is, intracellular changes according to a single action of an hTERT targeting ribozyme and a combination with radiation were confirmed under conditions of radiation alone and untreated GCV. The adenovirus was used with RZ-001 expressing ECRT-122T, and Ad-Mock without expressing a ribozyme was used as a control group ((a) of FIG. 16A).

The expression level of rH2AX was confirmed by staining with anti-rH2AX antibody 4 and 48 hours after irradiation and adenovirus infection. In untreated cells, an increase in rH2AX spots was confirmed 48 hours after RZ-001 infection, and thus it can be seen that genomic DNA instability increases due to a decrease in TERT expression due to RZ-001 infection. In the combined treatment cell line, an increase in rH2AX spot was confirmed at 4 hours after irradiation in all samples, including radiation-alone cells, and thus it can be seen that genomic DNA instability increases due to irradiation. It was confirmed that 48 hours after the combined treatment, genomic DNA repair was performed through a decrease in spots of rH2AX in cells treated with radiation alone, but the rH2AX spots were maintained in cells combined-treated with irradiation + RZ-001 infection, and thus it can be seen that the effect of genomic DNA instability persists due to the combined treatment with radiation + RZ-001 ((b) of FIG. 16A). Meanwhile, in the radiation + Ad-mock treated group, no increase or maintenance of rH2AX spots was observed, and it can be seen that the result is a specific phenomenon caused by ribozyme expression rather than a non-specific reaction due to adenovirus infection.

Meanwhile, the increase and maintenance of genomic DNA instability according to RZ-001 and irradiation were re-verified through observation of ATM expression levels activated under DNA damage conditions. Specifically, in the irradiation group, transiently activated p-ATM increased for 4 hours after irradiation and tended to return to its original state 48 hours after irradiation. However, in the radiation + RZ-001 combined group, the increase in p-ATM was maintained for up to 48 hours, similarly to rH2AX, and thus it can be seen that DNA damage was continuously maintained by the combined treatment of irradiation and RZ-001 ((c) of FIG. 16A).

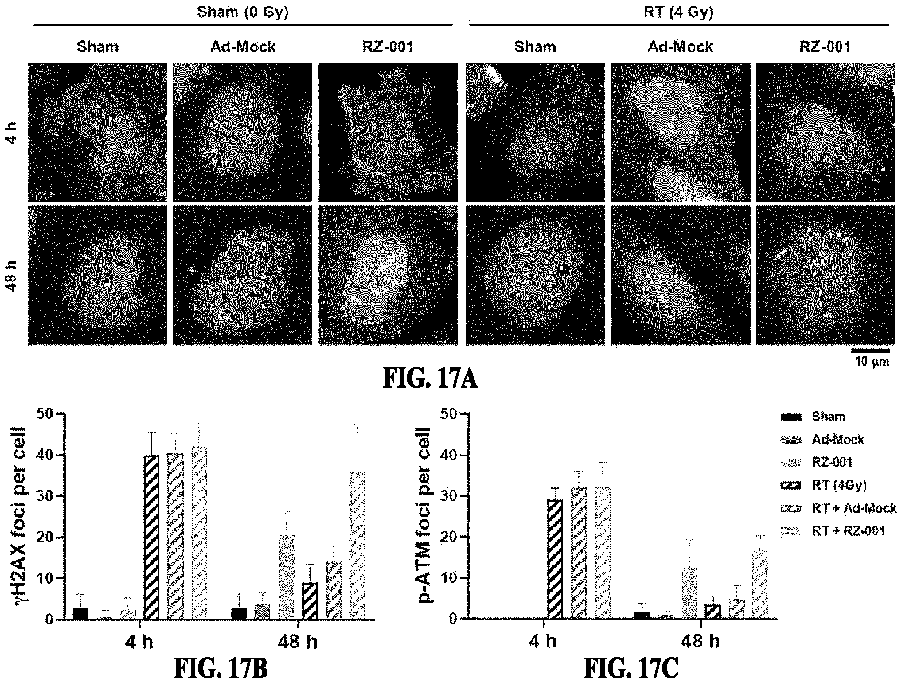

Meanwhile, an increase in rH2AX protein expression and the occurrence of apoptosis according to irradiation and RZ-001 treatment were reconfirmed through Western blot using an anti-rH2AX antibody and an anti-cleaved PARP antibody (FIG. 17). In the case of radiation-alone treatment, the expression of both rH2AX and cleaved-PARP proteins was not observed at both 24 and 48 hours. On the other hand, under the combined conditions of adenovirus-alone treatment and radiation, rH2AX protein expression increased at 48 hours, and cleaved-PARP protein expression, an indicator of apoptosis, also increased.

From the results, it can be seen that the combined treatment with RZ-001 and radiation causes and maintains cell DNA damage to cause cell apoptosis. In addition, in addition to inducing apoptosis by HSVtk + GCV, an anticancer mechanism inducing intracellular apoptosis was also confirmed according to hTERT targeting by RZ-001 itself.

As described above, specific parts of the present disclosure have been described in detail, and it will be apparent to those skilled in the art that these specific techniques are merely preferred embodiments, and the scope of the present disclosure is not limited thereto. Therefore, the substantial scope of the present disclosure will be defined by the appended claims and their equivalents.

### [Sequence List]

For reference, the following sequences are provided for further explanation of the present disclosure.

**[Table 4]**

| SEQ No. | Feature | Sequence (5'→3') |
|---|---|---|
| 1 | CMV promoter sequence | gacattgatt attgactagt tattaatagt aatcaattac ggggtcatta gttcatagcc catatatgga gttccgcgtt acataactta cggtaaatgg cccgcctggc tgaccgccca acgacccccg cccattgacg tcaataatga cgtatgttcc catagtaacg ccaataggga ctttccattg acgtcaatgg gtggagtatt tacggtaaac tgcccacttg gcagtacatc aagtgtatca tatgc |
| 2 | hTERT mRNA sequence | caggcagcgc tgcgtcctgc tgcgcacgtg ggaagccctg gccccggcca cccccgcgat gccgcgcgct ccccgctgcc gagccgtgcg ctccctgctg cgcagccact accgcgaggt gctgccgctg gccacgttcg tgcggcgcct ggggccccag ggctggcggc tggtgcagcg cggggacccg gcggctttcc gcgcgctggt ggcccagtgc ctggtgtgcg tgccctggga cgcacggccg ccccccgccg ccccctcctt ccgccaggtg tcctgcctga aggagctggt ggcccgagtg ctgcagaggc tgtgcgagcg cggcgcgaag aacgtgctgg ccttcggctt cgcgctgctg gacggggccc gcgggggccc ccccgaggcc ttcaccacca gcgtgcgcag ctacctgccc aacacggtga ccgacgcact gcgggggagc ggggcgtggg ggctgctgct gcgccgcgtg ggcgacgacg tgctggttca cctgctggca cgctgcgcgc tctttgtgct ggtggctccc agctgcgcct accaggtgtg cgggccgccg ctgtaccagc tcggcgctgc cactcaggcc cggcccccgc cacacgctag tggaccccga aggcgtctgg gatgcgaacg ggcctggaac catagcgtca gggaggccgg ggtccccctg ggcctgccag ccccgggtgc gaggaggcgc gggggcagtg ccagccgaag tctgccgttg cccaagaggc ccaggcgtgg cgctgcccct gagccggagc ggacgcccgt tgggcagggg tcctgggccc acccgggcag gacgcgtgga ccgagtgacc gtggtttctg tgtggtgtca cctgccagac ccgccgaaga agccacctct ttggagggtg cgctctctgg cacgcgccac tcccacccat ccgtgggccg ccagcaccac gcgggccccc catccacatc gcggccacca cgtccctggg acacgccttg tcccccggtg tacgccgaga ccaagcactt cctctactcc tcaggcgaca aggagcagct gcggccctcc ttcctactca gctctctgag gcccagcctg actggcgctc ggaggctcgt ggagaccatc tttctgggtt ccaggccctg gatgccaggg actccccgca ggttgccccg cctgccccag cgctactggc aaatgcggcc cctgtttctg gagctgcttg ggaaccacgc gcagtgcccc tacggggtgc tcctcaagac gcactgcccg ctgcgagctg cggtcacccc agcagccggt gtctgtgccc gggagaagcc ccagggctct gtggcggccc ccgaggagga ggacacagac ccccgtcgcc tggtgcagct gctccgccag cacagcagcc cctggcaggt gtacggcttc gtgcgggcct gcctgcgccg gctggtgccc ccaggcctct ggggctccag gcacaacgaa cgccgcttcc tcaggaacac caagaagttc atctccctgg ggaagcatgc caagctctcg |
| | | ctgcaggagc tgacgtggaa gatgagcgtg cgggactgcg cttggctgcg caggagccca ggggttggct gtgttccggc cgcagagcac cgtctgcgtg aggagatcct ggccaagttc ctgcactggc tgatgagtgt gtacgtcgtc gagctgctca ggtctttctt ttatgtcacg gagaccacgt ttcaaaagaa caggctcttt ttctaccgga agagtgtctg gagcaagttg caaagcattg gaatcagaca gcacttgaag agggtgcagc tgcgggagct gtcggaagca gaggtcaggc agcatcggga agccaggccc gccctgctga cgtccagact ccgcttcatc cccaagcctg acgggctgcg gccgattgtg aacatggact acgtcgtggg agccagaacg ttccgcagag aaaagagggc cgagcgtctc acctcgaggg tgaaggcact gttcagcgtg ctcaactacg agcgggcgcg gcgccccggc ctcctgggcg cctctgtgct gggcctggac gatatccaca gggcctggcg caccttcgtg ctgcgtgtgc gggcccagga cccgccgcct gagctgtact ttgtcaaggt ggatgtgacg ggcgcgtacg acaccatccc ccaggacagg ctcacggagg tcatcgccag catcatcaaa ccccagaaca cgtactgcgt gcgtcggtat gccgtggtcc agaaggccgc ccatgggcac gtccgcaagg ccttcaagag ccacgtctct accttgacag acctccagcc gtacatgcga cagttcgtgg ctcacctgca ggagaccagc ccgctgaggg atgccgtcgt catcgagcag agctcctccc tgaatgaggc cagcagtggc ctcttcgacg tcttcctacg cttcatgtgc caccacgccg tgcgcatcag gggcaagtcc tacgtccagt gccaggggat cccgcagggc tccatcctct ccacgctgct ctgcagcctg tgctacggcg acatggagaa caagctgttt gcggggattc ggcgggacgg gctgctcctg cgtttggtgg atgatttctt gttggtgaca cctcacctca cccacgcgaa aaccttcctc aggaccctgg tccgaggtgt ccctgagtat ggctgcgtgg tgaacttgcg gaagacagtg gtgaacttcc ctgtagaaga cgaggccctg ggtggcacgg cttttgttca gatgccggcc cacggcctat tcccctggtg cggcctgctg ctggataccc ggaccctgga ggtgcagagc gactactcca gctatgcccg gacctccatc agagccagtc tcaccttcaa ccgcggcttc aaggctggga ggaacatgcg tcgcaaactc tttggggtct tgcggctgaa gtgtcacagc ctgtttctgg atttgcaggt gaacagcctc cagacggtgt gcaccaacat ctacaagatc ctcctgctgc aggcgtacag gtttcacgca tgtgtgctgc agctcccatt tcatcagcaa gtttggaaga accccacatt tttcctgcgc gtcatctctg acacggcctc cctctgctac tccatcctga aagccaagaa cgcagggatg tcgctggggg ccaagggcgc cgccggccct ctgccctccg aggccgtgca gtggctgtgc caccaagcat tcctgctcaa gctgactcga caccgtgtca cctacgtgcc actcctgggg tcactcagga cagcccagac gcagctgagt cggaagctcc cggggacgac gctgactgcc ctggaggccg cagccaaccc ggcactgccc tcagacttca agaccatcct ggactgatgg ccacccgccc acagccaggc cgagagcaga caccagcagc cctgtcacgc cgggctctac gtcccaggga gggaggggcg gcccacaccc aggcccgcac cgctgggagt ctgaggcctg agtgagtgtt tggccgaggc ctgcatgtcc ggctgaaggc tgagtgtccg gctgaggcct gagcgagtgt ccagccaagg gctgagtgtc cagcacacct gccgtcttca cttccccaca ggctggcgct cggctccacc ccagggccag cttttcctca ccaggagccc ggcttccact ccccacatag gaatagtcca tccccagatt cgccattgtt cacccctcgc cctgccctcc tttgccttcc acccccacca tccaggtgga gaccctgaga aggaccctgg gagctctggg aatttggagt gaccaaaggt gtgccctgta cacaggcgag gaccctgcac ctggatgggg gtccctgtgg gtcaaattgg ggggaggtgc tgtgggagta aaatactgaa tatatgagtt tttcagtttt gaaaaaaa |
| 3 | hTERT targeting trans-splicing ribozyme | ggcaggaaaa gttatcaggc atgcacctgg tagctagtct ttaaaccaat agattgcatc ggtttaaaag gcaagaccgt caaattgcgg gaaaggggtc aacagccgtt cagtaccaag tctcagggga aactttgaga tggccttgca aagggtatgg taataagctg acggacatgg tcctaaccac gcagccaagt cctaagtcaa cagatcttct gttgatatgg atgcagttca cagactaaat gtcggtcggg gaagatgtat tcttctcata agatatagtc ggacctctcc ttaatgggag ctagcggatg aagtgatgca acactggagc |
| | | cgctgggaac taatttgtat gcgaaagtat attgattagt tttggagtac tcg |
| 4 | HSV-tk | atggcttcgt acccctgcca tcaacacgcg tctgcgttcg accaggctgc gcgttctcgc ggccatagca accgacgtac ggcgttgcgc cctcgccggc agcaagaagc cacggaagtc cgcctggagc agaaaatgcc cacgctactg cgggtttata tagacggtcc tcacgggatg gggaaaacca ccaccacgca actgctggtg gccctgggtt cgcgcgacga tatcgtctac gtacccgagc cgatgactta ctggcaggtg ctgggggctt ccgagacaat cgcgaacatc tacaccacac aacaccgcct cgaccagggt gagatatcgg ccggggacgc ggcggtggta atgacaagcg cccagataac aatgggcatg ccttatgccg tgaccgacgc cgttctggct cctcatatcg gggggggaggc tgggagctca catgccccgc ccccggccct caccctcatc ttcgaccgcc atcccatcgc cgccctcctg tgctacccgg ccgcgcgata ccttatgggc agcatgaccc cccaggccgt gctggcgttc gtggccctca tcccgccgac cttgcccggc acaaacatcg tgttgggggc ccttccggag gacagacaca tcgaccgcct ggccaaacgc cagcgccccg gcgagcggct tgacctggct atgctggccg cgattcgccg cgtttacggg ctgcttgcca atacggtgcg gtatctgcag ggcggcgggt cgtggcggga ggattgggga cagctttcgg ggacggccgt gccgccccag ggtgccgagc cccagagcaa cgcgggccca cgaccccata tcggggacac gttatttacc ctgtttcggg cccccgagtt gctggccccc aacggcgacc tgtacaacgt gtttgcctgg gccttggacg tcttggccaa acgcctccgt cccatgcacg tctttatcct ggattacgac caatcgcccg ccggctgccg ggacgccctg ctgcaactta cctccgggat ggtccagacc cacgtcacca cccccggctc cataccgacg atctgcgacc tggcgcgcac gtttgcccgg gagatggggg aggctaactg a |
| 5 | miR-122T | caaacaccat tgtcacactc ca |
| 6 | SD/SA sequence | agatctgaac tgaaaaacca gaaagttaac tggtaagttt agtctttttg tcttttattt caggtcccgg atccggtggt ggtgcaaatc aaagaactgc tcctcagtgg atgttgcctt tacttctagg cctgtacgga agtgttactt ctgctctaaa agctgcggaa ttgtacccag g |
| 7 | WPRE sequence | aatcaacctc tggattacaa aatttgtgaa agattgactg gtattcttaa ctatgttgct ccttttacgc tatgtggata cgctgcttta atgcctttgt atcatgctat tgcttcccgt atggctttca ttttctcctc cttgtataaa tcctggttgc tgtctcttta tgaggagttg tggcccgttg tcaggcaacg tggcgtggtg tgcactgtgt ttgctgacgc aacccccact ggttggggca ttgccaccac ctgtcagctc ctttccggga ctttcgcttt ccccctccct attgccacgg cggaactcat cgccgcctgc cttgcccgct gctggacagg ggctcggctg ttgggcactg acaattccgt ggtgttgtcg gggaaatcat cgtcctttcc ttggctgctc gcctgtgttg ccacctggat tctgcgcggg acgtccttct gctacgtccc ttcggccctc aatccagcgg accttccttc ccgcggcctg ctgccggctc tgcggcctct tccgcgtctt cgccttcgcc ctcagacgag tcggatctcc ctttgggccg cctccccgc |
| 8 | ribozyme antisense sequence | aaggccagca cgttcttcgc gccgcgctcg cacagcctct gcagcactcg ggccaccagc tccttcaggc aggacacctg gcggaaggag ggggcggcgg ggggcggccg tgcgtcccag ggcacgcaca ccaggcactg ggccaccagc gcgcggaaag ccgccgggtc cccgcgctgc accagccgcc agccctgggg ccccaggcgc cgcacgaacg tggccagcgg cagcacctcg cggtagtggc tgcgcagcag ggagcgcacg gctcggcagc ggggagcgcg cggcatcgcg ggggtggccg gggccagggc ttccca |
| 9 | Beta-globin poly(A) signal | aataaaggaa atttattttc attgcaatag tgtgttggaa ttttttgtgt ctctca |
| 10 | ribozyme forward primer | ttccggagga cagacacatc ga |
| 11 | ribozyme reverse primer | gcagataccg caccgtattg gc |

## Claims

1. A pharmaceutical composition for preventing or treating cancer, comprising a trans-splicing ribozyme expression vector targeting a cancer-specific gene sequence, a gene delivery system comprising the vector, or a ribozyme expressed from the vector, and combined with radiotherapy.

2. The pharmaceutical composition of claim 1, wherein the expression vector comprises a cytomegalovirus (CMV) promoter operably linked to the ribozyme gene,
a splicing donor/splicing acceptor sequence (SD/SA sequence) at a 5' end site of the ribozyme gene, and
a Woodchuck hepatitis virus posttranscriptional regulatory element (WPRE) at a 3' end site.

3. The pharmaceutical composition of claim 1, wherein the cancer-specific gene sequence is selected from the group consisting of telomerase reverse transcriptase (TERT) mRNA, alpha fetoprotein (AFP) mRNA, carcinoembryonic antigen (CEA) mRNA, prostate-specific antigen (PSA) mRNA, cytoskeleton-associated protein 2 (CKAP2) mRNA, and mutant Rat sarcoma (RAS) mRNA.

4. The pharmaceutical composition of claim 1, wherein the trans-splicing ribozyme comprises a nucleic acid sequence represented by SEQ ID NO: 3.

5. The pharmaceutical composition of claim 1, wherein the expression vector comprises a target gene linked to a 3' exon of the ribozyme gene.

6. The pharmaceutical composition of claim 5, wherein the target gene is an anti-cancer therapeutic gene or a reporter gene.

7. The pharmaceutical composition of claim 6, wherein the anti-cancer therapeutic gene is selected from the group consisting of a drug-sensitizing gene, a proapoptotic gene, a cytostatic gene, a cytotoxic gene, a tumor suppressor gene, an antigenic gene, a cytokine gene, and an anti-angiogenic gene.

8. The pharmaceutical composition of claim 7, wherein the drug-sensitizing gene is a Herpes Simplex Virus thymidine kinase (HSVtk) gene.

9. The pharmaceutical composition of claim 1, wherein the expression vector comprises a gene encoding a sequence complementary to part or all of microRNA-122 (miR-122) at a 3'-UTR end site of the ribozyme gene.

10. The pharmaceutical composition of claim 9, wherein the gene encoding the sequence complementary to miR-122 contains at least one copy of a base sequence represented by SEQ ID NO: 5.

11. The pharmaceutical composition of claim 1, wherein the cancer is at least one cancer selected from the group consisting of liver cancer, thyroid cancer, testicular cancer, bone cancer, glioblastoma, ovarian cancer, brain cancer, gallbladder cancer, biliary tract cancer, colon cancer, head and neck cancer, lymphoma, bladder cancer, leukemia, peritoneal cancer, small intestine cancer, esophageal cancer, renal pelvis cancer, kidney cancer, heart cancer, duodenal cancer, eye cancer, urethral cancer, breast cancer, stomach cancer, prostate cancer, uterine cancer, lung cancer, spinal tumor, pancreatic cancer, and melanoma.

12. The pharmaceutical composition of claim 1, wherein the cancer is cancer in which miR-122 is not substantially expressed in a cancer tissue.

13. The pharmaceutical composition of claim 12, wherein the cancer is liver cancer, and
the liver cancer is caused by at least one selected from the group consisting of hepatitis B virus, hepatitis C virus which reduces the expression of miR-122 in liver cancer tissue, alcohol, chronic hepatitis, cirrhosis, non-alcoholic fatty liver disease, aflatoxin, and family history.

14. The pharmaceutical composition of claim 1, wherein the pharmaceutical composition is administered simultaneously or sequentially with radiotherapy.

15. The pharmaceutical composition of claim 14, wherein the pharmaceutical composition is administered sequentially after radiotherapy 1 to 3 times.

16. The pharmaceutical composition of claim 15, wherein the pharmaceutical composition is repeatedly administered once a day, 1 to 3 times.

17. The pharmaceutical composition of claim 16, wherein the pharmaceutical composition is administered every other day when being administered twice or more.

18. A pharmaceutical composition for treating radiation-resistant cancer, comprising a trans-splicing ribozyme expression vector targeting a cancer-specific gene sequence, a gene delivery system comprising the vector, or a ribozyme expressed from the vector as an active ingredient.
